(19) Europäisches Patentamt
European Patent Office
Office européen des brevets

(11) **EP 4 293 360 A1**

(12) **EUROPEAN PATENT APPLICATION**
published in accordance with Art. 153(4) EPC

(43) Date of publication:
**20.12.2023 Bulletin 2023/51**

(21) Application number: **22752857.7**

(22) Date of filing: **14.02.2022**

(51) International Patent Classification (IPC):
**G01N 33/68** (2006.01)        **C07K 16/18** (2006.01)
**C12N 15/13** (2006.01)        **G01N 33/53** (2006.01)

(52) Cooperative Patent Classification (CPC):
**C07K 16/00; C07K 16/18; C12Q 1/06; G01N 33/53; G01N 33/68**

(86) International application number:
**PCT/JP2022/005590**

(87) International publication number:
**WO 2022/173035 (18.08.2022 Gazette 2022/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.02.2021 JP 2021021500**

(71) Applicants:
• **Canon Medical Systems Corporation Tochigi 324-0036 (JP)**
• **General Incorporated Association The Japan Multinational Trial Organization Nagoya-shi, Aichi 460-0002 (JP)**

(72) Inventors:
• **FUJITA, Masaaki Nagoya-shi, Aichi 460-0002 (JP)**

• **HUANG, Cheng-long Nagoya-shi, Aichi 460-0002 (JP)**
• **WADA, Hiromi Nagoya-shi, Aichi 460-0002 (JP)**
• **YASUDA, Yuki Otawara-shi, Tochigi 324-0036 (JP)**
• **SAKURAI, Yasuo Otawara-shi, Tochigi 324-0036 (JP)**

(74) Representative: **Marks & Clerk LLP 15 Fetter Lane London EC4A 1BW (GB)**

Remarks:
The complete document including Reference Table(s) and the Sequence Listing(s) can be downloaded from the EPO website

(54) **ADULT STILL'S DISEASE INSPECTION METHOD AND INSPECTION KIT**

(57)        The present disclosure relates to a method useful in the determination of adult Still's disease (ASD). More specifically, the present disclosure relates to:
(I) a method for testing adult Still's disease, including the following (1) and (2):
(1) measuring a denatured CRP concentration in a blood sample collected from a subject; and
(2) comparing the measured denatured CRP concentration and/or an index value based on the denatured CRP concentration with a reference value;

(II) a denatured CRP-specific antibody; and
(III) a kit for testing adult Still's disease.

FIG. 11

EP 4 293 360 A1

**Description**

Field

The present disclosure relates to a test method, a test kit, and the like for adult Still's disease.

Background

C-reactive proteins (CRPs) are generally used as an inflammation marker. It is suggested that there are CRPs in different conformations, that is, pentameric CRP (pCRP) that is a native CRP and a denatured CRP in which constituent factors are dissociated by denaturation of the pentameric CRP, typically monomeric CRP (mCRP), which play different physiological roles.

Since a clinical test reagent of CRP used as an index of inflammation is intended to measure the concentration of the native CRP and cannot specifically measure the concentration of the denatured CRP such as mCRP, it is not common to measure the concentration of the denatured CRP in clinical practice. As the concentration of the denatured CRP, the concentration of the denatured CRP in blood plasma from patients with acute myocardial infarction (20.96 ng/mL) (Non Patent Literature 5) and the concentration of the denatured CRP in blood plasma from patients with psoriasis, eczema and urticaria (skin-related autoimmune disease) (35.4 ng/mL, 30.0 ng/mL and 59.8 ng/mL, respectively) (Non Patent Literature 6) have been reported.

Adult Still's disease (ASD) is a disease having three symptoms of pyrexia, arthritis, and eruption as a cardinal sign. ASD includes ASD that transitions from Adult onset Still's disease (AOSD), a disease of a type that develops in adults, and systemic onset juvenile idiopathic arthritis (sJIA) that develops at a young age. ASD is likely to be confused with similar diseases presenting similar symptoms (Non Patent Literatures 1 to 3). Accordingly, the diagnosis of ASD is based on the diagnosis excluding similar diseases in accordance with the classification criteria of Yamaguchi et al. (Non Patent Literature 4). One of such similar diseases is vasculitis. However, in vasculitis, there may be cases showing only pyrexia and a high level of CRP. In such cases of vasculitis, differentiation from ASD is difficult.

Citation List

Non Patent Literatures

Non Patent Literature 1: Yayoi NAGAI, 2006, The Japanese Journal of Dermatology, 116(4): 429-436.
Non Patent Literature 2: Noboru Hagino, 2009, The Journal of the Japanese Society of Internal Medicine, 98(10):2 421-2431.
Non Patent Literature 3: Kohei Nagasawa, 2010, The Journal of the Japanese Society of Internal Medicine, 99(10): 2460-2466.
Non Patent Literature 4: Yamaguchi et al., 1992, The Journal of Rheumatology, 19: 424-430.
Non Patent Literature 5: Wang et al., Atherosclerosis, 2015, vol. 239, p. 343-349
Non Patent Literature 6: Zhang et al., Frontiers in Immunology, 2018, vol. 9, Article 511

Summary

Technical Problem

An object of the present disclosure is to provide a method useful in determining ASD.

Solution to Problem

As a result of intensive studies, the present inventors have found that a denatured CRP concentration and/or an index value (for example, CRP denaturation degree) based on the denatured CRP concentration are useful for the test for ASD (Example 5). Interestingly and unexpectedly, the denatured CRP concentration in blood samples from ASD patients was significantly higher than previously reported denatured CRP concentrations known to the present inventors, and ASD showed the highest CRP denaturation degree as compared to other inflammatory diseases (Example 5).

Based on the above findings, the present inventors have found that ASD can be tested and differentiated based on the denatured CRP concentration and/or an index value based thereon.

That is, the present disclosure relates to the following and the like.

In one embodiment, disclosed is a method for testing adult Still's disease, comprising the following (1) and (2):

(1) measuring a denatured CRP concentration in a blood sample collected from a subject; and
(2) comparing the measured denatured CRP concentration and/or an index value based on the denatured CRP concentration with a reference value.

In another embodiment, disclosed is a kit for testing adult Still's disease, comprising a denatured CRP-specific antibody.

In a specific embodiment, the subject in the above method and kit may be a subject contracting an inflammatory disease or a subject suspected of contracting an inflammatory disease.

In a specific embodiment, the subject in the above method and kit may be a subject contracting a disease other than an infectious disease or a subject suspected of contracting a disease other than an infectious disease.

In a specific embodiment, the above method and kit may be a method for differentiating adult Still's disease from vasculitis, rheumatoid arthritis, polymyalgia rheumatica, or systemic lupus erythematosus.

In a specific embodiment, the index value utilized in the above method and kit is a value calculated from a relational expression between the denatured CRP concentration and a CRP value.

In a specific embodiment, the value calculated from the relational expression between the denatured CRP concentration and the CRP value, which is utilized in the above method and kit may be a value calculated from a relative relational expression between the denatured CRP concentration and the CRP value.

In a specific embodiment, the subject in the above method and kit may be a subject whose CRP value is measured.

In a specific embodiment, the subject in the above method and kit may be a subject whose exhibits a CRP value higher than the reference value.

In a specific embodiment, the subject in the above method and kit may be a subject whose CRP value is not measured, and the method and kit may further comprises a step of measuring the CRP value in a blood sample collected from the subject, or an antibody to CRP.

In a specific embodiment, the above method and kit may correspond to a test further comprising selecting and subjecting a subject whose measured CRP value is higher than the reference value to the comparing step.

In a specific embodiment, the above method and kit are for measuring the denatured CRP concentration using a denatured CRP-specific antibody.

In a specific embodiment, the above method and kit are for measuring the denatured CRP concentration using the denatured CRP-specific antibody selected from the group consisting of the following (1) to (3), or a combination thereof:

(1) an antibody comprising the following (1a) and (1b) :

(1a) an antibody heavy chain containing a variable region comprising an amino acid sequence having an identity of 90% or more to the amino acid sequence consisting of the amino acid residues at positions 20 to 133 in the amino acid sequence of SEQ ID NO: 4, and
(1b) an antibody light chain containing a variable region comprising an amino acid sequence having an identity of 90% or more to the amino acid sequence consisting of the amino acid residues at positions 20 to 131 in the amino acid sequence of SEQ ID NO: 9;

(2) an antibody comprising the following (2a) and (2b) :

(2a) an antibody heavy chain containing a variable region comprising an amino acid sequence having an identity of 90% or more to the amino acid sequence consisting of the amino acid residues at positions 20 to 141 in the amino acid sequence of SEQ ID NO: 14, and
(2b) an antibody light chain containing a variable region comprising an amino acid sequence having an identity of 90% or more to the amino acid sequence consisting of the amino acid residues at positions 21 to 133 in the amino acid sequence of SEQ ID NO: 19; and

(3) an antibody comprising the following (3a) and (3b) :

(3a) an antibody heavy chain containing a variable region comprising an amino acid sequence having an identity of 90% or more to the amino acid sequence consisting of the amino acid residues at positions 20 to 141 in the amino acid sequence of SEQ ID NO: 24, and
(3b) an antibody light chain containing a variable region comprising an amino acid sequence having an identity of 90% or more to the amino acid sequence consisting of the amino acid residues at positions 21 to 133 in the amino acid sequence of SEQ ID NO: 29.

In another embodiment, disclosed is a denatured CRP-specific antibody selected from the group consisting of the following (1) to (3):

(1) an antibody comprising the following (1a) and (1b) :

(1a) an antibody heavy chain containing a variable region comprising an amino acid sequence having an identity of 90% or more to the amino acid sequence consisting of the amino acid residues at positions 20 to 133 in the amino acid sequence of SEQ ID NO: 4, and
(1b) an antibody light chain containing a variable region comprising an amino acid sequence having an identity of 90% or more to the amino acid sequence consisting of the amino acid residues at positions 20 to 131 in the amino acid sequence of SEQ ID NO: 9;

(2) an antibody comprising the following (2a) and (2b) :

(2a) an antibody heavy chain containing a variable region comprising an amino acid sequence having an identity of 90% or more to the amino acid sequence consisting of the amino acid residues at positions 20 to 141 in the amino acid sequence of SEQ ID NO: 14, and
(2b) an antibody light chain containing a variable region comprising an amino acid sequence having an identity of 90% or more to the amino acid sequence consisting of the amino acid residues at positions 21 to 133 in the amino acid sequence of SEQ ID NO: 19; and

(3) an antibody comprising the following (3a) and (3b) :

(3a) an antibody heavy chain containing a variable region comprising an amino acid sequence having an identity of 90% or more to the amino acid sequence consisting of the amino acid residues at positions 20 to 141 in the amino acid sequence of SEQ ID NO: 24, and
(3b) an antibody light chain containing a variable region comprising an amino acid sequence having an identity of 90% or more to the amino acid sequence consisting of the amino acid residues at positions 21 to 133 in the amino acid sequence of SEQ ID NO: 29.

Advantageous Effects of Invention

According to the present disclosure, it is possible to test for ASD and to differentiate ASD from vasculitis, rheumatoid arthritis, polymyalgia rheumatica or systemic lupus erythematosus, and to assist clinical diagnosis.

Brief Description of Drawings

FIG. 1 is a diagram illustrating an amino acid sequence of human CRP (GenBank Accession number: AAL48218), and a neutralizing epitope in human CRP recognized by a neutralizing antibody specific to human denatured CRP (SEQ ID NO: 2) (Positions 99 to 108 in the Full-length amino acid sequence. Positions 81 to 90 in signal removal sequence).

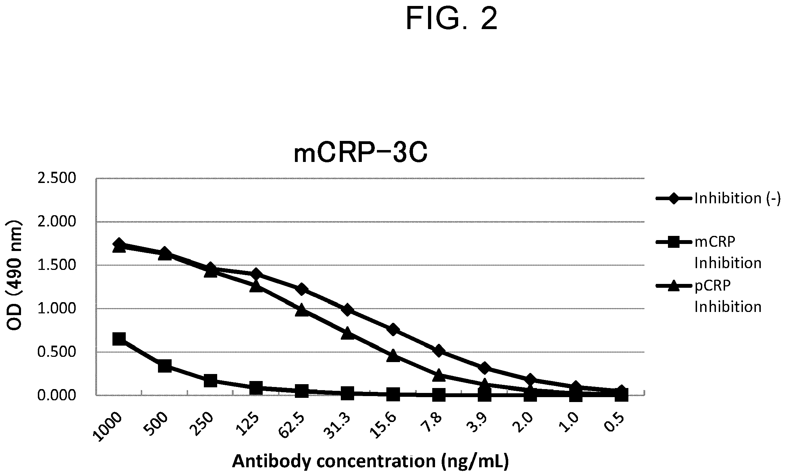

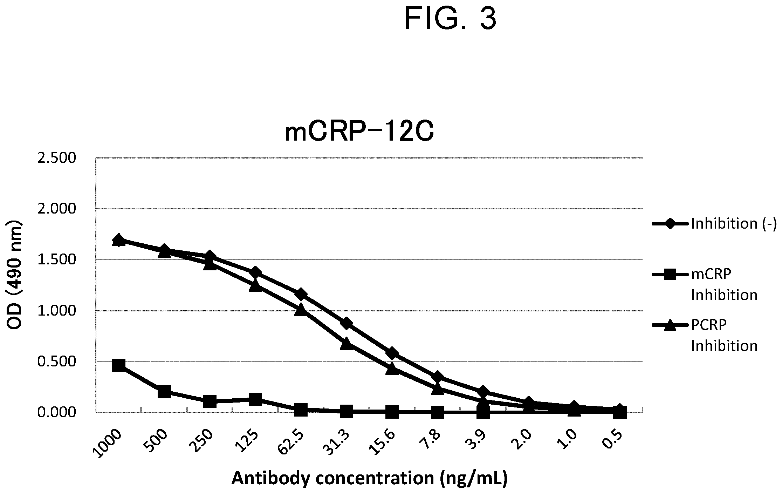

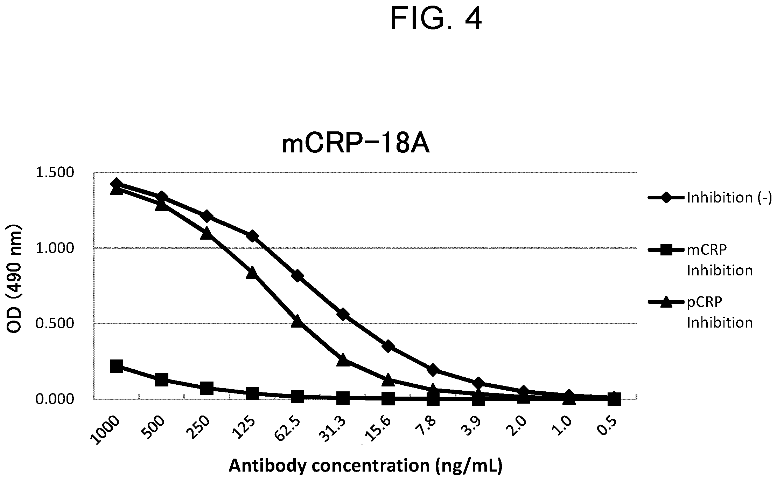

FIG. 2 is a diagram illustrating a result of analyzing the IgG purified from a culture supernatant obtained by culturing hybridoma mCRP-3C by inhibition ELISA. Inhibition (-): an experimental result (control) in the case of using a solution obtained by pre-incubating the IgG purified from the culture supernatant of the hybridoma with a solution not containing both human monomeric CRP (mCRP) and human pentamer (pCRP) (synonymous with simple "CRP"); mCRP inhibition: an experimental result in the case of using a solution obtained by pre-incubating the IgG purified from the culture supernatant of the hybridoma with a solution containing an excess amount of human mCRP; pCRP inhibition: an experimental result in the case of using a solution obtained by pre-incubating the IgG purified from the culture supernatant of the hybridoma with a solution containing an excess amount of human pCRP (the same applies to FIGS. 3 and 4.).
FIG. 3 is a diagram illustrating a result of analyzing the IgG purified from a culture supernatant obtained by culturing hybridoma mCRP-12C by inhibition ELISA.
FIG. 4 is a diagram illustrating a result of analyzing the IgG purified from a culture supernatant obtained by culturing hybridomas mCRP-3C, mCRP-12C, and mCRP-18A by inhibition ELISA.

FIG. 5 is a diagram illustrating a signal sequence in a heavy chain of an antibody (3C) purified from a culture supernatant obtained by culturing hybridoma mCRP-3C, and CDR1 (SEQ ID NO: 5), CDR2 (SEQ ID NO: 6) and CDR3 (SEQ ID NO: 7).

FIG. 6 is a diagram illustrating a signal sequence in a light chain of an antibody (3C) purified from a culture supernatant obtained by culturing hybridoma mCRP-3C, and CDR1 (SEQ ID NO: 10), CDR2 (SEQ ID NO: 11) and CDR3 (SEQ ID NO: 12).

FIG. 7 is a diagram illustrating a signal sequence in a heavy chain of an antibody (12C) purified from a culture supernatant obtained by culturing hybridoma mCRP-12C, and CDR1 (SEQ ID NO: 15), CDR2 (SEQ ID NO: 16) and CDR3 (SEQ ID NO: 17).

FIG. 8 is a diagram illustrating a signal sequence in a light chain of an antibody (12C) purified from a culture supernatant obtained by culturing hybridoma mCRP-12C, and CDR1 (SEQ ID NO: 20), CDR2 (SEQ ID NO: 21) and CDR3 (SEQ ID NO: 22).

FIG. 9 is a diagram illustrating a signal sequence in a heavy chain of an antibody (18A) purified from a culture supernatant obtained by culturing hybridoma mCRP-18A, and CDR1 (SEQ ID NO: 25), CDR2 (SEQ ID NO: 26) and CDR3 (SEQ ID NO: 27).

FIG. 10 is a diagram illustrating a signal sequence in a light chain of an antibody (18A) purified from a culture supernatant obtained by culturing hybridoma mCRP-18A, and CDR1 (SEQ ID NO: 30), CDR2 (SEQ ID NO: 31) and CDR3 (SEQ ID NO: 32).

FIG. 11 is a diagram illustrating a ratio of a denatured CRP concentration to a CRP concentration in each of patients with adult-onset Still's disease (AOSD), rheumatoid arthritis (RA), polymyalgia rheumatica (PMR), and vasculitis.

Description of Embodiments

(Test Method for Adult Still's Disease)

The present disclosure relates to a method for testing adult Still's disease (ASD).

The test method includes the following (1) and (2) :

(1) measuring a denatured CRP concentration in a blood sample collected from a subject; and
(2) comparing the measured denatured CRP concentration and/or an index value based on the denatured CRP concentration with a reference value.

In the test method, when the measured denatured CRP concentration is higher than the reference value, it can be used as an index of adult Still's disease. Further, in a case where the index value based on the denatured CRP concentration is a value calculated to be high by reflecting the level of the denatured CRP concentration, when the index value based on the measured denatured CRP concentration is higher than the reference value, it can be used as an index of adult Still's disease. Furthermore, in a case where the index value based on the denatured CRP concentration is a value calculated to be low by reflecting the level of the denatured CRP concentration, when the index value based on the measured denatured CRP concentration is lower than the reference value, it can be used as an index of adult Still's disease.

Examples of the subject include mammals (for example, primates such as humans and monkeys; rodents such as mice, rats, and hamsters; and pets or working animals such as dogs, cats, rabbits, sheep, goats, donkeys, horses, cattle, and pigs). Preferably, the subject is a human.

In one embodiment, the subject may be a subject contracting an inflammatory disease or a subject suspected of contracting an inflammatory disease. Such a subject can be tested for the possibility of contracting ASD as an inflammatory disease. Examples of the inflammatory disease to be differentiated from ASD include vasculitis, arthritis (for example, rheumatoid arthritis, psoriatic arthritis, osteoarthritis, and juvenile arthritis), systemic lupus erythematosus, polymyalgia rheumatica, infectious diseases (for example, viruses, microorganisms, and parasites), myocardial infarction, and skin-related inflammatory disease (for example, psoriasis, eczema, and urticaria). According to the above method, ASD can be differentiated from such inflammatory diseases by appropriately setting conditions such as a reference value.

In another embodiment, the subject may be a subject contracting a disease other than an infectious disease or suspected of contracting a disease other than an infectious disease. An infectious disease may often be easy to diagnose depending on its symptoms. Further, since infectious diseases can be definitely diagnosed by identifying a pathogen (for example, viruses and microorganisms) (for example, identification by gene/genome detection), ASD is often easily distinguished from infectious diseases. Thus, in test for ASD, a subject contracting an inflammatory disease other than an infectious disease or a subject suspected of contracting an inflammatory disease other than an infectious disease may be tested.

In yet another embodiment, the subject may be a subject for whom differentiation of ASD from vasculitis, rheumatoid arthritis, polymyalgia rheumatica, or systemic lupus erythematosus is desired.

In certain embodiments, the subject may be a subject whose CRP value has been measured. In this case, a subject exhibiting a CRP value higher than the reference value can be subjected to the test method. In the clinical test of CRP, although the reference may vary depending on the inspection organization and country, a concentration of less than 3 $\mu$g/mL (0.3 mg/dL) is often employed as one indicator of the CRP concentration in the normal range. Accordingly, such a reference value may be a concentration of 3 $\mu$g/L or more, preferably a concentration of 5 $\mu$g/mL or more, more preferably a concentration of 10 $\mu$g/mL or more, still more preferably a concentration of 20 $\mu$g/mL or more, particularly preferably a concentration of 30 $\mu$g/mL or more, a concentration of 40 $\mu$g/mL or more, or a concentration of 50 $\mu$g/mL or more. Such a reference value may also be at a concentration of 100 $\mu$g/mL or less, preferably at a concentration of 90 $\mu$g/mL or less, more preferably at a concentration of 80 $\mu$g/mL or less, even more preferably at a concentration of 70 $\mu$g/mL or less, particularly preferably at a concentration of 60 $\mu$g/mL or less. More specifically, such reference value may be 3 to 100 $\mu$g/mL, preferably 5 to 90 $\mu$g/mL, more preferably 10 to 80 $\mu$g/mL, even more preferably 20 to 70 $\mu$g/mL, and particularly preferably 30 to 60 $\mu$g/mL, 40 to 60 $\mu$g/mL, or 50 to 60 $\mu$g/mL.

Further, when the CRP value has already been measured in the subject, the redundant measurement of the CRP value can be omitted, and thus the index value (for example, a value calculated from a relational expression between a denatured CRP concentration and a CRP value as described later) based on the denatured CRP concentration can be easily calculated.

In another certain embodiment, the subject may be a subject whose CRP value has not been measured. In this case, the test method may further include a step of measuring the CRP value in a blood sample collected from the subject. By such a step, it is possible to select a subject whose measured CRP value is higher than the reference value and to calculate an index value (for example, a value calculated from a relational expression between a denatured CRP concentration and a CRP value as described later) based on the denatured CRP concentration after measuring the CRP value.

Examples of the blood sample include whole blood (peripheral blood), blood plasma, serum, and samples obtained by treating these. Such treatments include centrifugation, fractionation, extraction, filtration, precipitation, refrigeration, freezing, and heating, for example.

The concentration is the content (for example, values defined as relative values such as g/L and molar concentration) of the target substance per unit solution amount. Accordingly, the concentration may be evaluated as the absolute value (for example, grams and moles) of the target substance in a certain amount of solution.

The measurement of the denatured CRP concentration can be performed by any method capable of measuring the amount of the denatured CRP. Examples of such a method include immunoassays and chromatography (for example, gel filtration chromatography). Examples of the immunoassay include enzyme immunoassay (EIA) (for example, direct competition ELISA, indirect competition ELISA, and sandwich ELISA), radioimmunoassay (RIA), fluorescence immunoassay (FIA), immunochromatography, luminescence immunoassay, blotting (for example, western blotting), and latex agglutination. In the immunoassay, any antibody such as a polyclonal antibody, a monoclonal antibody, or an engineered antibody (for example, a single chain antibody or an Fc fragment) can be used.

When the measurement of the denatured CRP concentration is performed by an immunoassay, the immunoassay can be performed using an antibody specific to the denatured CRP.

The "denatured CRP-specific antibody" refers to an antibody having higher binding ability to a denatured CRP (preferably, human monomeric CRP) than to a native CRP.

The "human monomeric CRP" (mCRP) refers to a monomeric protein of human CRP (signal sequence may be removed) having the amino acid sequence of SEQ ID NO: 1 (GenBank Accession number: AAL48218) or a naturally occurring variant thereof (for example, natural variant or SNP or haplotype).

The "native CRP" refers to a pentameric protein composed of the human monomeric CRP.

The "denatured CRP" refers to a denatured CRP in which constituent factors are dissociated by denaturation of the native CRP. Examples of the denatured CRP include monomeric CRP (mCRP), dimeric CRP, trimeric CRP, and tetrameric CRP, and combinations of one or more kinds (for example, two kinds, three kinds, and four kinds) thereof.

In the measurement of the denatured CRP concentration by an immunoassay, two or more kinds of antibodies may be used. When two or more kinds of antibodies are used in the measurement of the denatured CRP concentration (for example, sandwich assay), a set of antibodies containing two kinds of antibodies specific to a denatured CRP (allogeneic or xenogeneic, preferably xenogeneic) can be used. Alternatively, a set of antibodies containing a combination of one kind of antibody specific to a denatured CRP and one kind of antibody against a native CRP may be used. This is because if at least one kind of antibody is a denatured CRP-specific antibody, the denatured CRP can be measured. Preferably, from the viewpoint of improving the specificity to a denatured CRP, a set of antibodies containing two kinds of antibodies specific to a denatured CRP may be used. For example, when two or more kinds of antibodies are used, at least one kind of antibody can be used as a solid phase antibody, and at least one kind of antibody can be used as a labeled antibody.

The antibody specific to a denatured CRP can be obtained by: 1) producing a hybridoma that produces an antibody against a denatured CRP by using a denatured CRP (preferably, human monomeric CRP) as an antigen, 2) selecting a hybridoma that produces an antibody having higher binding ability for monomeric CRP than the binding ability for pentameric CRP by using a denatured CRP and pentameric CRP as antigens, and 3) isolating the antibody from the culture supernatant of the selected hybridoma, or isolating the antibody from a culture of transformed cells incorporating a gene of the antibody produced by the hybridoma. The antibody specific to the denatured CRP can be confirmed by checking that the test antibody has a higher binding ability to the denatured CRP than the binding ability to the pentameric CRP using the denatured CRP and the pentameric CRP as antigens (see inhibition ELISA described in the Examples). Alternatively, since a known antibody (for example, Non Patent Literatures 3 and 4) is present as a denatured CRP-specific antibody, such a known antibody can be used as a denatured CRP-specific antibody.

Preferably, the denatured CRP-specific antibody may be an antibody described later.

In one embodiment, the measured denatured CRP concentration is compared to a reference value. In ASD, it has been demonstrated that the denatured CRP concentration in blood samples is high (Example 5). Accordingly, when the measured denatured CRP concentration is higher than the reference value as a result of comparing the measured denatured CRP concentration with the reference value, it can be determined that the subject may be contracting ASD.

In another embodiment, an index value based on the measured denatured CRP concentration is compared to a reference value. Such an index value is not particularly limited as long as it is an index capable of reflecting the level of the denatured CRP concentration. Further, since it has been found that the CRP value in a blood sample is high in ASD, and that the CRP denaturation degree that is the relative ratio of the denatured CRP concentration to the CRP value (denatured CRP concentration/CRP value) in ASD is high (Example 5), the index value can be set on the basis of such a relative ratio.

The CRP value can be measured by any method capable of measuring the amount of CRP. Examples of such a method include immunoassays and chromatography (for example, gel filtration chromatography) as described above.

When the CRP value is measured by an immunoassay, the immunoassay can be performed using an antibody against CRP. A clinical test reagent of CRP containing an antibody against CRP, which is used as an index of inflammation, is usually intended to measure the concentration of the native CRP, but may not be checked or disclosed for binding to a denatured CRP. This is considered to be because, although a native CRP has been conventionally widely used as a classical inflammation marker, the presence and significance of a denatured CRP are conventionally unknown, and there is no motivation to check binding to a denatured CRP, or there is no or low need to disclose binding to a denatured CRP. Accordingly, it is considered that the clinical test reagent of CRP contains an antibody capable of binding to a native CRP and at least a part of a denatured CRP depending on the type thereof, and the total concentration of the native CRP and at least a part of the denatured CRP is measured in some cases. Accordingly, the CRP value may be a native CRP concentration or a total concentration of the native CRP concentration and the concentration of at least a part of the denatured CRP. In the measurement of CRP, both a clinical test reagent of CRP capable of measuring only the concentration of the native CRP and a clinical test reagent of CRP capable of measuring the total concentration of the native CRP and at least a part of a denatured CRP can be used. Further, since antibodies capable of binding to a native CRP and a denatured CRP are known (for example, JP 2004-189665 A), such known antibodies may be used for measurement.

In a preferred embodiment, the index value based on the denatured CRP concentration is a value calculated from a relational expression between the denatured CRP concentration and the CRP value. As described above, this is because it has been found that in ASD, the denatured CRP concentration is high, the CRP value is high, and the relative ratio of the denatured CRP concentration to the CRP value is high. The index value based on the denatured CRP concentration varies depending on factors such as the type of the subject to be differentiated (for example, a healthy subject, a patient with an inflammatory disease, a patient with vasculitis, a patient with rheumatoid arthritis, a patient with polymyalgia rheumatica, and a patient with systemic lupus erythematosus), and desired diagnostic sensitivity and diagnostic specificity, and can be appropriately set according to the purpose of the test.

Examples of the value calculated from the relational expression between the denatured CRP concentration and the CRP value include a value calculated from the relative relational expression between the denatured CRP concentration and the CRP value, and a value calculated from the non-relative relational expression between the denatured CRP concentration and the CRP value.

Examples of the value calculated from the relative relational expression between the denatured CRP concentration and the CRP value include a value calculated from the following expression.

(A) (denatured CRP concentration)$^{n1}$/(CRP value)$^{m1}$
[where n1 is an arbitrary exponent (briefly an integer of 1) and m1 is an arbitrary exponent (briefly an integer of 1).]
(B) (CRP value)$^{n2}$/(denatured CRP concentration)$^{m2}$
[where n2 is an arbitrary exponent (briefly an integer of 1) and m2 is an arbitrary exponent (briefly an integer of 1)]

The value calculated from the relational expression (A) is a value calculated to be high by reflecting the level of the denatured CRP concentration. Accordingly, when the value calculated from the relational expression (A) is used as the index value, it can be determined that there is a possibility that the subject is contracting ASD when the index value is higher than the reference value.

Meanwhile, the value calculated from the relational expression (B) is a value calculated to be low by reflecting the level of the denatured CRP concentration. Accordingly, when the value calculated from the relational expression of (B) is used as the index value, it can be determined that there is a possibility that the subject is contracting ASD when the index value is lower than the reference value.

Briefly, the value calculated from the relative relational expression between the denatured CRP concentration and the CRP value may be a value calculated from the following expression.

(A') Ratio of denatured CRP concentration to CRP value (denatured CRP concentration/CRP value) (that is, CRP denaturation degree)
(B') Ratio of CRP value to denatured CRP concentration (CRP value/denatured CRP concentration)

Examples of the value calculated from the non-relative relational expression between the denatured CRP concentration and the CRP value include a value calculated from the following expression.

$$(C) \quad (\text{denatured CRP concentration})^{n5} \times (\text{CRP value})^{m5}$$

[where n5 is an arbitrary exponent (most simply an integer of 1) and m5 is an arbitrary exponent (most simply an integer of 1)]

The value calculated from the relational expression (C) is a value calculated to be high by reflecting the level of the denatured CRP concentration. Accordingly, when the value calculated from the relational expression of (C) is used as the index value, it can be determined there is a possibility that the subject is contracting ASD when the index value is higher than the reference value.

Briefly, the value calculated from the non-relative relational expression between the denatured CRP concentration and the CRP value may be a value calculated from the following expression.

$$(C') \quad \text{Denatured CRP concentration} \times \text{CRP value}$$

As the value calculated from the relational expression between the denatured CRP concentration and the CRP value, a value calculated from the relative relational expression between the denatured CRP concentration and the CRP value is preferable, a value calculated from the above expression (A) or (B) is more preferable, and a value calculated from the above expression (A) (that is, CRP denaturation degree) is particularly preferable.

The reference value of the denatured CRP concentration or the index value based thereon may vary depending on the type of subject to be differentiated (for example, a healthy subject, a patient with an inflammatory disease, a patient with vasculitis, a patient with rheumatoid arthritis, a patient with polymyalgia rheumatica, and a patient with systemic lupus erythematosus) and factors such as desired diagnostic sensitivity and diagnostic specificity. For example, as such a reference value, a value within a normal range for the denatured CRP concentration in a blood sample of a comparative subject (for example, a healthy subject, a patient with an inflammatory disease, a patient with vasculitis, a patient with rheumatoid arthritis, a patient with polymyalgia rheumatica, and a patient with systemic lupus erythematosus) or the index value based on the denatured CRP concentration can be appropriately used. Furthermore, as such a reference value, for example, a cut-off value, an average value of the denatured CRP concentration in a blood sample derived from a comparative subject or an index value based thereon, or a value obtained by adding a certain value to the average value (for example, mean + SD or mean + 2SD) may be used. The cut-off value can be appropriately set by those skilled in the art. Such a reference value may also vary depending on factors such as the type of method for measuring the denatured CRP concentration and the CRP value (for example, kinds of antibodies used in an immunoassay), and whether the CRP value is used as a cut-off value for selecting a subject based on the CRP value or not.

More specifically, as an example of the denatured CRP concentration or the index value based on the denatured CRP concentration, the reference value of the denatured CRP concentration may be a value of 200 ng/mL or more, preferably a value of 300 ng/mL or more, more preferably a value of 400 ng/mL or more, and still more preferably a value of 500 ng/mL or more (Tables 5 and 7), for example. Further, an upper limit value may be provided for convenience as a reference value of the denatured CRP concentration. For example, such an upper limit value may be a value of 3,000 ng/mL or less, a value of 2,500 ng/mL or less, a value of 2,000 ng/mL or less, or a value of 1,500 ng/mL or less.

Alternatively, when the CRP denaturation degree (denatured CRP concentration/CRP value) is indicated as an index value based on the denatured CRP concentration, and the ratio is calculated in different concentration units of the denatured CRP concentration (ng/mL) and the CRP value (μg/mL), the reference value of the CRP denaturation degree may be a value of 2.2 or more, preferably a value of 2.3 or more, more preferably a value of 2.4 or more, and still more preferably a value of 2.6 or more (Tables 5 and 7). Further, an upper limit value may be provided for convenience as a reference value of the CRP denaturation degree. For example, such an upper limit value may be a value of 100 or less, a value of 50 or less, a value of 20 or less, a value of 10 or less, a value of 8 or less, or a value of 6 or less. As a matter of course, when ratios are calculated in the same concentration unit (ng/mL), the above reference value is a value of $10^{-3}$.

A plurality of the denatured CRP concentrations or index values based on the denatured CRP concentrations may be used in combination. For example, a combination of a denatured CRP concentration and one or more index values based on the denatured CRP concentration, and a combination of two or more index values based on the denatured CRP concentration can be used as the combination. In such a case, the value of each reference value can be set gently.

Denatured CRP concentrations in ASD patients are prominently high among inflammatory diseases in which CRP values are high. Thus, according to the methods described above, ASD (for example, AOSD) patients can be differentiated from a certain subject (for example, a healthy subject, a patient with an inflammatory disease, a patient with vasculitis, a patient with rheumatoid arthritis, a patient with polymyalgia rheumatica, and a patient with systemic lupus erythematosus). Thus, the methods described above are useful for test for ASD (for example, AOSD).

(Antibody)

The present disclosure relates to a denatured CRP-specific antibody and an invention related thereto.
The antibody may be an antibody selected from the group consisting of the following (1) to (3):

(1) An antibody comprising the following (1a) and (1b) :

(1a) an antibody heavy chain containing a variable region comprising an amino acid sequence having an identity of 90% or more to an amino acid sequence consisting of amino acid residues at positions 20 to 133 in the amino acid sequence of SEQ ID NO: 4, and
(1b) an antibody light chain containing a variable region comprising an amino acid sequence having an identity of 90% or more to an amino acid sequence consisting of amino acid residues at positions 20 to 131 in the amino acid sequence of SEQ ID NO: 9;

(2) an antibody comprising the following (2a) and (2b) :

(2a) an antibody heavy chain containing a variable region comprising an amino acid sequence having an identity of 90% or more to an amino acid sequence consisting of amino acid residues at positions 20 to 141 in the amino acid sequence of SEQ ID NO: 14, and
(2b) an antibody light chain containing a variable region comprising an amino acid sequence having an identity of 90% or more to an amino acid sequence consisting of amino acid residues at positions 21 to 133 in the amino acid sequence of SEQ ID NO: 19; and

(3) an antibody comprising the following (3a) and (3b) :

(3a) an antibody heavy chain containing a variable region comprising an amino acid sequence having an identity of 90% or more to an amino acid sequence consisting of amino acid residues at positions 20 to 141 in the amino acid sequence of SEQ ID NO: 24, and
(3b) an antibody light chain containing a variable region comprising an amino acid sequence having an identity of 90% or more to an amino acid sequence consisting of amino acid residues at positions 21 to 133 in the amino acid sequence of SEQ ID NO: 29.

Preferably, the antibody may be an antibody selected from the group consisting of the following (1) to (3).

(1) An antibody comprising the following (1a) and (1b) :

(1a) an antibody heavy chain comprising an amino acid sequence having an identity of 90% or more to an amino acid sequence consisting of amino acid residues at positions 20 to 469 in the amino acid sequence of SEQ ID NO: 4, and

(1b) an antibody light chain comprising an amino acid sequence having an identity of 90% or more to an amino acid sequence consisting of amino acid residues at positions 20 to 238 in the amino acid sequence of SEQ ID NO: 9;

(2) an antibody comprising the following (2a) and (2b) :

(2a) an antibody heavy chain comprising an amino acid sequence having an identity of 90% or more to an amino acid sequence consisting of amino acid residues at positions 20 to 465 in the amino acid sequence of SEQ ID NO: 14, and
(2b) an antibody light chain comprising an amino acid sequence having an identity of 90% or more to an amino acid sequence consisting of amino acid residues at positions 21 to 240 in the amino acid sequence of SEQ ID NO: 19; and

(3) an antibody comprising the following (3a) and (3b) :

(3a) an antibody heavy chain comprising an amino acid sequence having an identity of 90% or more to an amino acid sequence consisting of amino acid residues at positions 20 to 476 in the amino acid sequence of SEQ ID NO: 24, and
(3b) an antibody light chain comprising an amino acid sequence having an identity of 90% or more to an amino acid sequence consisting of amino acid residues at positions 21 to 240 in the amino acid sequence of SEQ ID NO: 29.

Alternatively, the antibody may be an antibody containing: (a) an antibody heavy chain comprising an amino acid sequence having an identity of 90% or more to the amino acid sequence of SEQ ID NO: 61, SEQ ID NO: 63, SEQ ID NO: 65, SEQ ID NO: 67, SEQ ID NO: 69, or SEQ ID NO: 71; and (b) an antibody light chain comprising an amino acid sequence having an identity of 90% or more to the amino acid sequence of SEQ ID NO: 73, SEQ ID NO: 75, SEQ ID NO: 77, or SEQ ID NO: 79.

The identity described above may be 91% or more, 92% or more, 93% or more, 94% or more, 95% or more, 96% or more, 97% or more, 98% or more, or 99% or more. The identity can be calculated by an algorithm blastp. More specifically, the identity can be calculated using Scoring Parameters (Matrix: BLOSUM62; Gap Costs: Existence = 11 Extension = 1; Compositional Adjustments: Conditional compositional score matrix adjustment) set by default in an algorithm blastp provided from National Center for Biotechnology Information (NCBI).

Further, an amino acid sequence having a desired identity to a target amino acid sequence can be identified by mutation of a desired number of amino acid residues to the target amino acid sequence. The mutation of an amino acid residue is one, two, three, or four kinds of mutations selected from the group consisting of deletion, substitution, addition, and insertion of an amino acid residue. The mutation of the amino acid residue may be introduced into one region in the amino acid sequence, or may be introduced into a plurality of different regions. For example, when the target amino acid sequence is composed of 400 or more amino acid residues, an amino acid sequence having an identity of 90% or more to the target amino acid sequence is allowed to have 40 or less amino acid residue mutations to the target amino acid sequence, and an amino acid sequence having an identity of 95% or more to the target amino acid sequence is allowed to have 20 or less amino acid residue mutations to the target amino acid sequence. Further, when the target amino acid sequence is composed of 300 or more amino acid residues, in an amino acid sequence having an identity of 90% or more to the target amino acid sequence, 30 or less amino acid residue mutations to the target amino acid sequence are allowed, and in an amino acid sequence having an identity of 95% or more to the target amino acid sequence, 15 or less amino acid residue mutations to the target amino acid sequence are allowed. Furthermore, when the target amino acid sequence is composed of 200 or more amino acid residues, an amino acid sequence having an identity of 90% or more to the target amino acid sequence is allowed to have 20 or less amino acid residue mutations to the target amino acid sequence, and an amino acid sequence having an identity of 95% or more to the target amino acid sequence is allowed to have 10 or less amino acid residue mutations to the target amino acid sequence. Further, when the target amino acid sequence is composed of 100 or more amino acid residues, an amino acid sequence having an identity of 90% or more to the target amino acid sequence is allowed to have 10 or less amino acid residue mutations to the target amino acid sequence, and an amino acid sequence having an identity of 95% or more to the target amino acid sequence is allowed to have 5 or less amino acid residue mutations to the target amino acid sequence.

Examples of the isotype of the human denatured CRP-specific antibody include IgG (for example, IgG1, IgG2, IgG3, and IgG4), IgM, IgA (for example, IgA1 and IgA2), IgD, and IgE, preferably IgG or IgM, and more preferably IgG.

The present disclosure also relates to a polynucleotide comprising a nucleotide sequence encoding a human denatured CRP-specific antibody. Examples of the polynucleotide include DNA and RNA, and DNA is preferable.

The present disclosure also relates to an expression vector containing the polynucleotide and a promoter operably linked thereto. Examples of the promoter include a promoter of a gene highly expressed in a desired host cell, and a virus-derived promoter. The promoter may be a constitutive promoter or an inducible promoter.

The expression vector may also further include elements such as terminators that function in the host cell, ribosome binding sites, and drug resistance genes. Examples of the drug resistance gene include resistance genes against drugs such as tetracycline, ampicillin, kanamycin, hygromycin, and phosphinothricin.

The expression vector may also further contain a region that allows for homologous recombination with the genome of the host cell for homologous recombination with the genomic DNA of the host cell. For example, the expression vector may be designed such that the expression unit contained therein is located between a pair of homologous regions (for example, homology arms, loxP, and FRT, homologous to specific sequences in the genome of the host cell). The genomic region (target of the homologous region) of the host cell into which the expression unit is to be introduced is not particularly limited, and may be a locus of a gene having a high expression level in the host cell.

The expression vector may be a plasmid, a viral vector, a phage, or an artificial chromosome. The expression vector may also be an integrative or non-integrative vector. An integrative vector may be a vector of a type that is entirely integrated into the genome of a host cell. Alternatively, an integrative vector may be a vector of a type in which only a portion (for example, expression units) thereof is integrated into the genome of a host cell. The expression vector may further be a DNA vector or an RNA vector (for example, a retrovirus).

The present disclosure also relates to a transformed cell that contains an expression unit containing the polynucleotide and a promoter operably linked thereto.

The "expression unit" refers to the smallest unit that contains a certain polynucleotide to be expressed as a protein and a promoter operably linked thereto, and allows transcription of the polynucleotide and the production of the protein encoded by the polynucleotide. The expression unit may further contain elements such as a terminator, a ribosome binding site, and a drug resistance gene. The expression unit may be DNA or RNA, and is preferably DNA. The expression unit may also be an expression unit that contains one polynucleotide to be expressed as a protein and a promoter operably linked thereto (that is, an expression unit allowing expression of monocistronic mRNA) or an expression unit that contains a plurality of polynucleotides to be expressed as a protein (for example, a polynucleotide encoding a heavy chain and a polynucleotide encoding a light chain) and a promoter operably linked thereto (that is, an expression unit allowing expression of polycistronic mRNA). The expression unit may be contained in the genomic region at one or more (for example, one, two, three, four, or five) different positions. Specific forms of the expression unit contained in the non-genomic region include plasmids, viral vectors, phages, and artificial chromosomes, for example. The promoter is the same as described above.

The transformed cell can be produced by any method known in the art. For example, the transformed cell can be produced by a method using an expression vector (for example, a competent cell method and an electroporation method) or a genome editing technique. If the expression vector is an integrative vector that results in homologous recombination with the genomic DNA of the host cell, the expression unit can be integrated into the genomic DNA of the host cell by transformation. Meanwhile, when the expression vector is a non-integrative vector that does not undergo homologous recombination with the genomic DNA of the host cell, the expression unit is not incorporated into the genomic DNA of the host cell by transformation, and can exist independently of the genomic DNA in the host cell in the state of the expression vector. Alternatively, according to a genome editing technology (for example, CRISPR/Cas system and Transcription Activator-Like Effector Nucleases (TALEN)), it is possible to incorporate an expression unit into the genomic DNA of a host cell. Examples of the host cell of the transformed cell include eukaryotic cells [for example, animal cells (for example, mammalian cells), plant cells, and eukaryotic microorganisms] and prokaryotic cells (for example, prokaryotic microorganisms).

(Test Kit)

The present disclosure also relates to a test kit including a denatured CRP-specific antibody.

Details of the denatured CRP-specific antibody are as described above.

In one embodiment, the kit is a test kit for ASD.

In another embodiment, the kit may further include an antibody against CRP or a clinical test reagent of CRP. That is, the kit may be a disease test kit including a combination of a denatured CRP-specific antibody and an antibody against CRP or a clinical test reagent of CRP. As far as the present inventors have grasped, it has been found for the first time that the ratio of the denatured CRP concentration that can be measured by a denatured CRP-specific antibody to the CRP to the CRP value that can be measured by an antibody against CRP or a clinical test reagent of CRP (an example of the ratio is the CRP denaturation degree) is useful for diagnosis of a disease. That is, although a motivation to use such a combination occurs for the first time according to the research results by the present inventors, there is no motivation to combine these in the prior art that does not focus on such a ratio. Thus, the use of such a combination in the diagnosis of a disease is not readily conceivable from the prior art.

In certain embodiments, the kit may include two or more kinds of antibodies for measurement of denatured CRP concentrations (for example, sandwich immunoassay). As such two or more kinds of antibodies, a set of antibodies containing two kinds of antibodies specific to the denatured CRP (allogeneic or xenogeneic, preferably xenogeneic) can be used. Alternatively, a set of antibodies containing a combination of one kind of antibody specific to a denatured CRP and one kind of antibody against a native CRP may be used. This is because if at least one kind of antibody is a denatured CRP-specific antibody, the denatured CRP can be measured. Preferably, from the viewpoint of improving the specificity to a denatured CRP, a set of antibodies containing two kinds of antibodies specific to a denatured CRP may be used. For example, when two or more kinds of antibodies are used, at least one kind of antibody can be used as a solid phase antibody, and at least one kind of antibody can be used as a labeled antibody.

In another certain embodiment, the kit is a kit for an immunoassay. The kit is preferably a kit for ELISA, and more preferably a kit for sandwich ELISA. Thus, the kit may include:

(1) a first antibody specific to a denatured CRP (solid phase antibody);
(2) a second antibody specific to a denatured CRP (labeled antibody); and
(3) a solid phase.

In still another certain embodiment, the kit is an immunoassay kit capable of measuring a CRP value in addition to a denatured CRP concentration. The kit further includes one or more kinds of antibodies against CRP. The kit is preferably a kit for ELISA, and more preferably a kit for sandwich ELISA. Thus, the kit may include:

(1) a first antibody specific to a denatured CRP (solid phase antibody);
(2) a second antibody specific to a denatured CRP (labeled antibody);
(3) (a) a combination of a first antibody against CRP and a second antibody against CRP, or (b) a CRP clinical diagnostic; and
(4) a solid phase.

As the antibody, any antibody such as a polyclonal antibody, a monoclonal antibody, or an engineered antibody (for example, a single chain antibody or an Fc fragment) can be used. The antibody may be immobilized on a solid phase. Examples of the solid phase include a plate, a support, a membrane, and particles (for example, magnetic particles and latex particles). For example, when the kit is an ELISA kit, the kit may be configured to include a solid phase on which a solid phase antibody is immobilized and a labeled antibody. Further, in a case where the kit is a kit other than the ELISA kit, for example, a kit for immunochromatography, the kit may be configured so as to include a support on which a solid phase antibody and a labeled antibody to the target substance are immobilized.

The kit may also include an auxiliary means. Examples of such an auxiliary means include a buffer, a stabilizer, an enzyme used for labeling an antibody and a substrate of the enzyme, and a container for pretreating blood (for example, a blood collection tube).

The kit can be suitably used in the above method, for example.

Examples

The present disclosure will be described in more detail with reference to the following examples, but the present disclosure is not limited to the following examples.

Example 1: Preparation of human denatured CRP-specific monoclonal antibody

(1) Preparation of antigen (human mCRP)

Human pentameric CRP (C-Reactive Protein Human Pleural Fluid, Lee biosolutions, catalogue number: 140-11) (human pCRP) was treated in 8 M Urea/10 mM EDTA at 37°C for 2 hours to prepare a human monomeric CRP (human mCRP) solution. After preparation, dialysis was performed in a 10 mM phosphate buffer (pH 7.4) and 15 mM NaCl using a dialysis tube. The dialyzed solution was transferred to a dialysis tube, then centrifuged at 3,500 xg at 4°C, and concentrated.

(2) Immunization of mice with antigen (human mCRP)

The antigen (human mCRP) concentration was prepared to 200 μg/0.5 mL, and the same amount of adjuvant was added to prepare an emulsion using a glass syringe. Freund's complete adjuvant (FCA, DIFCO, USA) was used as an adjuvant only at the time of initial immunization, and Freund's incomplete adjuvant (FICA, DIFCO, USA) was used for the second and subsequent immunizations. After four times of immunizations, an increase in antibody titer was checked, and final immunization (i.p./50 $\mu$g/animal) was performed.

The obtained emulsion was injected subcutaneously and intradermally into mice using a 27G injection needle. Thereafter, a total of four times of immunizations were performed every 7 days, and a small amount of blood was collected from the tail vein 7 days after the four times of immunizations. A diluted antiserum was added to an immunoplate on which human mCRP was immobilized, and the titer of the antiserum was checked by anti-mouse IgG-HRP detection. Specifically, the titer of the antiserum was checked by the following solid phase ELISA.

(3) Solid Phase ELISA

As materials for solid phase ELISA, the following were used.

(a) Plate: Immunoplate for ELISA (96 well) manufactured by Nunc
(b) Substrate: O.P.D tablet (SIGMA)
(c) Solid-phase buffer: 0.1 M carbonate buffer, pH 9.5 (IBL)
(d) Antiserum dilution buffer: 1% BSA, 0.05% Tween-20 (Kanto Chemical Co., Inc.) in PBS
(e) Labeled antibody dilution buffer: 1% BSA, 0.05% Tween-20 in PBS
(f) Washing buffer: 0.05% Tween-20 in 0.1 M phosphate buffer
(g) Substrate buffer: $K_2HPO_4$-citrate buffer (pH 5.1)
(h) Reaction stop solution: 1 mol/L-$H_2SO_4$ (Wako Pure Chemical Industries, Ltd.)
(i) Solid phase protein: human mCRP or BSA (control)

The solid phase ELISA was performed by the following procedure.

(a) The protein was immobilized on a plate by standing at 50 ng/well (50 $\mu$L/well) for 16 hours at 4°C.
(b) The solid phase protein was washed twice with Dulbecco's PBS (D-PBS) (200 $\mu$L/well).
(c) Blocking was performed by adding 200 $\mu$L of D-PBS containing 0.1% BSA and 0.05% $NaN_3$ to each well and leaving it at 4°C overnight.
(d) Each well was washed twice with the washing buffer.
(e) The sample (antiserum) was diluted with a dilution buffer, and the diluted solution was added to each well (50 $\mu$L/well) and left at 37°C for 30 minutes.
(f) Each well was washed four or more times with the washing buffer.
(g) Anti-mouse IgG (y-specific)-HRP, a labeled antibody, was added to each well (50 $\mu$L/well) and left at 37°C for 30 minutes.
(h) Each well was washed four or more times with the washing buffer.
(i) A chromogenic reaction was started by adding a substrate solution (400 $\mu$g/mL) to each well (100 $\mu$L/well), and the reaction was allowed to proceed for 15 minutes at room temperature in the dark.
(j) After 15 minutes, the reaction was stopped by addition of 1 mol/L sulfuric acid (100 $\mu$L/well), and absorbance (490 nm) was measured.

(4) Preparation of hybridoma that produces human denatured CRP-specific antibody

Spleen cells or lymph node cells collected from a mouse in which a significant increase in titer to human mCRP was observed by the solid phase ELISA were cell-fused with myeloma cells (X-63.Ag8 653 (ATCC)) using polyethylene glycol (PEG) (19 pieces of 96 well plates). Hybridomas were selectively cultured using a HAT (hypoxanthine, aminopterin, and thymidine) selective medium. Thereafter, the colony formation of the hybridoma was observed, and screening by the above-described solid phase ELISA (solid phase antigen: human mCRP) was performed using a supernatant dilution of the culture of the hybridoma as a sample, and 36 positive clones were selected.

(5) Inhibition ELISA

Next, hybridomas that produce human denatured CRP-specific antibodies having a ability of binding to human mCRP were selected by inhibition ELISA.

In the inhibition ELISA, a solid phase ELISA (solid phase antigen: human mCRP) as described above was performed using (i) a solution obtained by pre-incubating the culture supernatant of the hybridoma with a solution containing an excess amount of human mCRP, (ii) a solution obtained by pre-incubating the culture supernatant of the hybridoma with a solution containing an excess amount of human pCRP, and (iii) a solution (control) obtained by pre-incubating the culture supernatant of the hybridoma with a solution not containing both of human mCRP and human pCRP as samples. In such an inhibition ELISA, the culture supernatant of the hybridoma that produces the anti-human mCRP antibody having the ability of specifically binding to human mCRP does not bind to the solid phase antigen (human mCRP) because it binds to human mCRP in the solution in (i), and therefore a signal is not substantially detected. Meanwhile, in (ii), since such a culture supernatant does not bind to human pCRP in the solution and thus can bind to a solid phase antigen (human mCRP), a sufficient signal is detected. Further, in (iii), such a culture supernatant can bind to a solid phase antigen (human mCRP) because human mCRP and human pCRP are not present in the solution, and thus a sufficient signal is detected.

Specifically, the inhibition ELISA was performed as follows.

(a) Human mCRP was adjusted to 40 μg/mL with a dilution buffer (Solution a1). Alternatively, human pCRP was adjusted to 40 μg/mL with a dilution buffer (Solution a2).

(b) The culture supernatant was diluted 2-fold with a dilution buffer (solution 2).

(c) Each 50 μL of Solution a1 and Solution 2 were mixed, and the antibody in the culture supernatant and human mCRP were reacted at 4°C for 16 hours or more. In addition, 50 μL each of Solution a2 and Solution 2 were mixed, and the antibody in the culture supernatant and human pCRP were reacted at 4°C for 16 hours or more.

(d) The subsequent procedures were similar to the procedures of (a) to (j) described above for the solid phase ELISA. The solution obtained in (c) of the inhibition ELISA was used as the sample used in (e) of the solid phase ELISA. The results are shown in Table 1.

[Table 1]

| Table 1. Results of inhibition ELISA for 36 positive clones | | | |
|---|---|---|---|
| | (i) mCRP | (ii) pCRP | (iii) control |
| 1 | 0.001 | 0.203 | 0.303 |
| 2 | 0.007 | -0.002 | 0.023 |
| 3 | 0.043 | 1.245 | 1.390 |
| 4 | 0.000 | -0.002 | 0.009 |
| 5 | 0.014 | 0.357 | 0.414 |
| 6 | 0.031 | 0.237 | 0.258 |
| 7 | 0.036 | 1.311 | 1.533 |
| 8 | 0.024 | 0.010 | 0.019 |
| 9 | 0.001 | 0.002 | 0.007 |
| 10 | 0.021 | 0.046 | 0.027 |
| 11 | 0.067 | 0.103 | 0.103 |
| 12 | 0.826 | 2.197 | 2.175 |
| 13 | 0.068 | 1.809 | 1.977 |
| 14 | 0.039 | 1.020 | 1.347 |
| 15 | 0.004 | 0.205 | 0.394 |
| 16 | 0.022 | 0.556 | 0.945 |
| 18 | 0.921 | 2.446 | 2.291 |
| 19 | 2.024 | 2.366 | 2.293 |
| 20 | 0.094 | 1.882 | 2.007 |
| 21 | 0.220 | 2.243 | 2.129 |
| 22 | -0.002 | 0.048 | 0.205 |
| 24 | -0.002 | -0.006 | 0.003 |
| 25 | 0.005 | 0.081 | 0.182 |
| 26 | 0.013 | 0.047 | 0.142 |

(continued)

| Table 1. Results of inhibition ELISA for 36 positive clones | | | |
|---|---|---|---|
| | (i) mCRP | (ii) pCRP | (iii) control |
| 28 | 0.712 | 2.297 | 2.227 |
| 29 | 0.071 | 2.152 | 2.062 |
| 30 | 0.117 | 2.085 | 2.098 |
| 32 | 0.021 | 0.466 | 0.713 |
| 33 | -0.001 | 0.093 | 0.234 |
| 34 | 0.004 | 0.192 | 0.411 |
| 35 | 0.045 | 1.409 | 1.671 |
| 36 | 0.180 | 2.151 | 2.123 |

As a result, in many of the 36 positive clones, sample (i) showed a smaller signal value than samples (ii) and (iii) did. This indicates that many clones produce anti-human mCRP antibodies with the ability to specifically bind to human mCRP. Among these clones, seven kinds of clones (3, 12, 18, 19, 21, 35, and 36) in which signals were not substantially detected in (i) while sufficient signals were detected in (ii) and (iii) were selected. It is believed that these seven kinds of clones can produce excellent human denatured CRP-specific antibodies having the ability to bind to human mCRP.

(6) Primary cloning of hybridomas and subclass identification

The selected seven kinds of clones were primary cloned by a limiting dilution method. After two weeks, screening was performed by an EIA method to select positive wells. The positive wells were selected for each 3 wells (A, B, and C), the supernatant was collected, and solid-phase ELISA and inhibition ELISA were performed. As a result, good antibody reactivity was not observed in one kind of clone, but good antibody reactivity was observed in three kinds of subclones (A, B, and C) corresponding to six kinds of clones (clone numbers 3, 12, 18, 19, 21, and 35). In the subsequent experiments, these subclones (3C, 12C, 18A, 19C, 21A, and 35A) were used.

Next, subclass check by sandwich ELISA was performed using one culture supernatant of each clone, and the results were as follows.

(a) mCRP-3C: mouse IgG2b, κ

(b) mCRP-12C: mouse IgG1, κ

(c) mCRP-18A: mouse IgG2a, κ

(d) mCRP-19C: mouse IgG1, κ

(e) mCRP-21A: mouse IgG2a, κ

(f) mCRP-35A: mouse IgG2a, κ

(7) IgG purification

IgG was purified from the culture supernatant obtained by culturing six kinds of hybridomas (3C, 12C, 18A, 19C, 21A, and 35A) by a conventional method, and the binding ability to mCRP was analyzed.
Materials and instruments used for purification and analysis of IgG are as follows.

(a) Protein A column
(b) Binding buffer (glycine-NaCl, pH 8.9)
(c) Elution buffer (citric acid, pH 4.0)
(d) Regeneration buffer (citric acid, pH 2.0)

(e) D-PBS
(f) Absorbance monitor-280 nm (ATTO)
(g) AKTA System (GE Healthcare)
(h) Analytical column for AKTA (GE Healthcare)

Purification and analysis of IgG were performed as follows.

(Purification of IgG)

(a) The culture supernatant was filtered at 0.45 $\mu$m, subjected to salting-out with ammonium sulfate, and then diluted with the binding buffer.
(b) The resultant was added to the Protein A column equilibrated with the binding buffer.
(c) The column was sufficiently washed with the binding buffer to remove contaminant proteins.
(d) The resultant is subjected to IgG elution with the elution buffer. A neutralization buffer in an amount of about 1/10 of the recovered liquid was previously placed in a recovery container to neutralize the pH of the eluate.
(e) The eluate was dialyzed under refrigeration in PBS.
(f) IgG was collected and filtered at 0.22 $\mu$m.
(g) The OD of 280 nm was measured to calculate the IgG concentration.
(h) The mixture was concentrated to about 5 mg/mL by an ultrafiltration method, filtered at 0.22 $\mu$m, and then dispensed and cryopreserved.

(Analysis of IgG)

(a) The absorbance at 280 nm was measured, and the protein concentration was calculated with $A_{280}$ = 1.382 as 1 mg/mL.
(b) The purity was checked by gel filtration analysis with AKTA FPLC.
(c) The titer was checked by the solid phase ELISA described above.

As a result, it was confirmed that all of the IgGs purified from the culture supernatant obtained by culturing the six kinds of hybridomas had binding ability to human mCRP.

Example 2: Secondary screening of hybridomas capable of producing human denatured CRP-specific monoclonal antibodies

The subclones (mCRP-3C, mCRP-12C, and mCRP-18A) of the hybridoma obtained in Example 1 were secondarily cloned by a limiting dilution method. Cell culture and purification and analysis of IgG were performed in the same manner as in Example 1.
As a result, when the titer was checked by the solid phase ELISA described above, it was confirmed that all of the IgGs purified from the culture supernatant obtained by culturing the three kinds of hybridomas (mCRP-3C, mCRP-12C, and mCRP-18A) subjected to the secondary cloning have binding ability to human mCRP.
Further, when IgGs purified from the culture supernatant obtained by culturing these hybridomas were analyzed by the inhibition ELISA described in Example 1 (5), it was confirmed that these IgGs were human denatured CRP-specific monoclonal antibodies having the ability of binding to human mCRP (FIGS. 2 to 4).

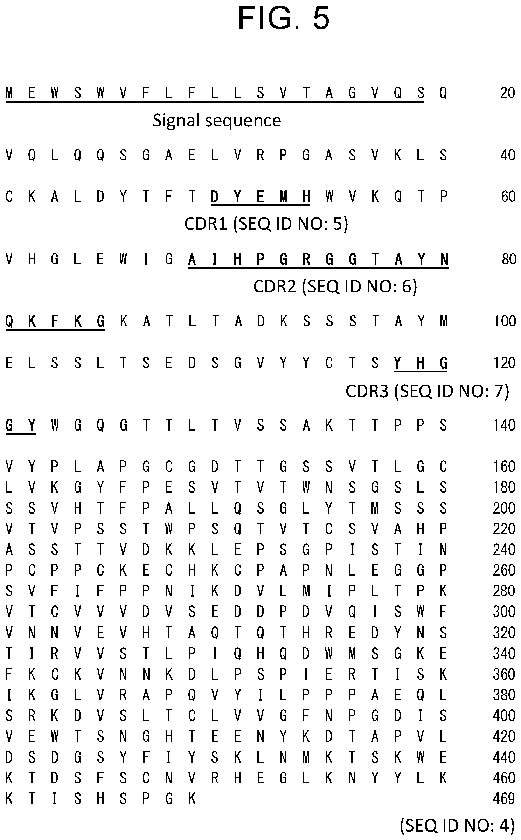

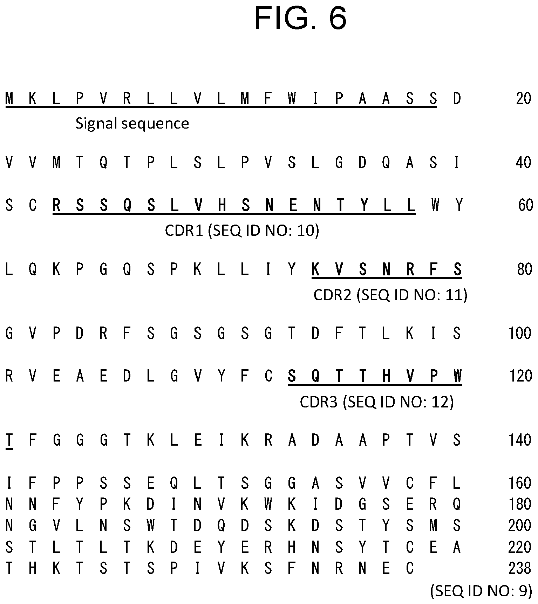

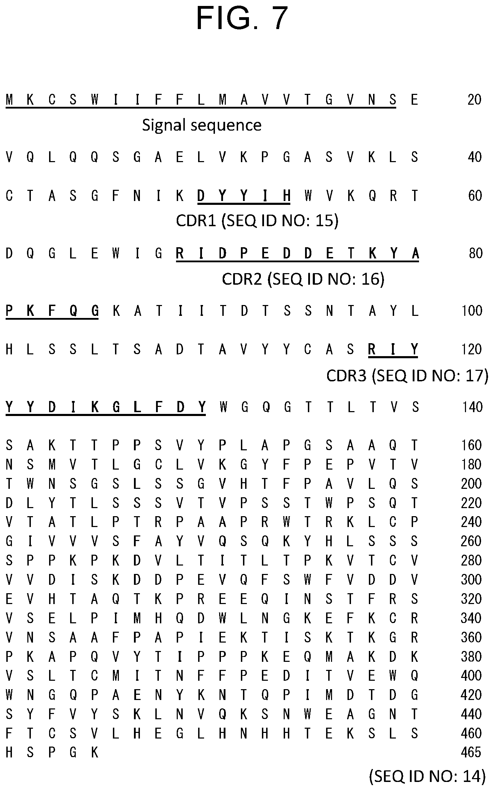

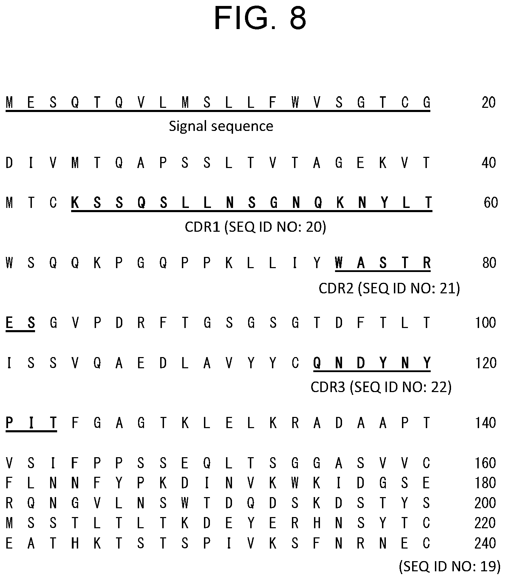

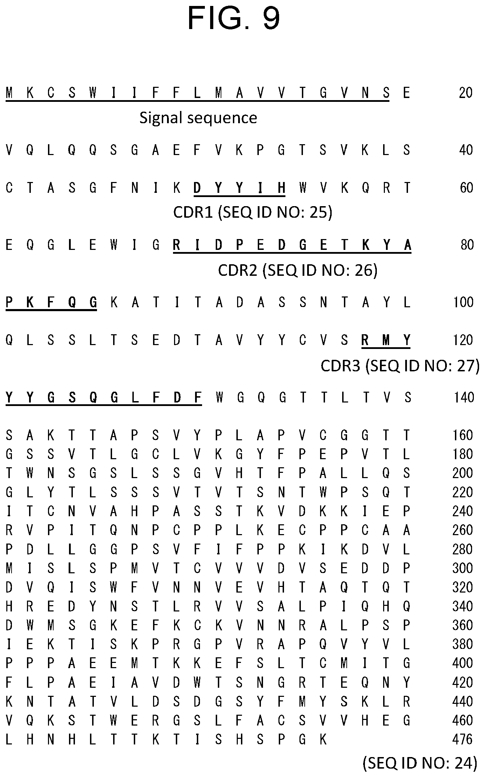

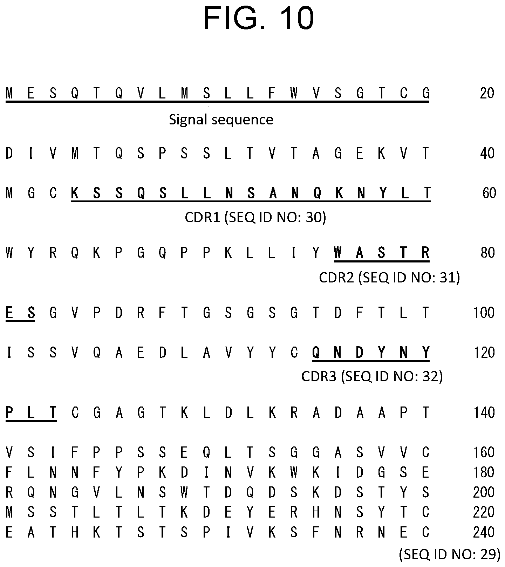

Example 3: Structural analysis of human denatured CRP-specific monoclonal antibody

mRNA was extracted from the three kinds of hybridomas (mCRP-3C, mCRP-12C, and mCRP-18A) secondarily screened in Example 2, and cDNA was prepared using a reverse transcriptase. A 5'-RACE (rapid amplification of cDNA ends) method was performed using primers specific to murine heavy chain and light chain to determine the N-terminal nucleotide sequences of the heavy chains and light chains. Full- cDNAs of a heavy chain and a light chain were cloned into an expression vector using a translation initiation methionine peripheral sequence of an N-terminal nucleotide sequence as an N-terminal primer. The CDRs and variable regions were determined according to the definition of Kabat et al. utilizing IgBLAST from NCBI.
As a result, the human denatured CRP-specific monoclonal antibody produced by each hybridoma had the structural characteristics shown in Table 2 below (see also FIGS. 5 to 10).

[Table 2]

| Table 2. Structures of human denatured CRP-specific monoclonal antibodies produced by various hybridomas | | |
|---|---|---|
| | Heavy chain | Light chain |
| mCRP-3C | Full-length nucleotide sequence: SEQ ID NO: 3<br>Full-length amino acid sequence: SEQ ID NO: 4<br>CDR1: DYEMH (SEQ ID NO: 5)<br><br>CDR2: AIHPGRGGTAYNQKFKG (SEQ ID NO: 6)<br>CDR3: YHGGY (SEQ ID NO: 7) | Full-length nucleotide sequence: SEQ ID NO: 8<br>Full-length amino acid sequence: SEQ ID NO: 9<br>CDR1: RSSQSLVHSNENTYLL (SEQ ID NO: 10)<br>CDR2: KVSNRFS (SEQ ID NO: 11)<br><br>CDR3: SQTTHVPWT (SEQ ID NO: 12) |
| mCRP-12C | Full-length nucleotide sequence: SEQ ID NO: 13<br>Full-length amino acid sequence: SEQ ID NO: 14<br>CDR1: DYYIH (SEQ ID NO: 15)<br><br>CDR2: RIDPEDDETKYAPKFQG (SEQ ID NO: 16)<br>CDR3: RIYYYDIKGLFDY (SEQ ID NO: 17) | Full-length nucleotide sequence: SEQ ID NO: 18<br>Full-length amino acid sequence: SEQ ID NO: 19<br>CDR1: KSSQSLLNSGNQKNYLT (SEQ ID NO: 20)<br>CDR2: WASTRES (SEQ ID NO: 21)<br><br>CDR3: QNDYNYPIT (SEQ ID NO: 22) |
| mCRP-18A | Full-length nucleotide sequence: SEQ ID NO: 23<br>Full-length amino acid sequence: SEQ ID NO: 24<br>CDR1: DYYIH (SEQ ID NO: 25)<br><br>CDR2: RIDPEDGETKYAPKFQG (SEQ ID NO: 26)<br>CDR3: RMYYYGSQGLFDF (SEQ ID NO: 27) | Full-length nucleotide sequence: SEQ ID NO: 28<br>Full-length amino acid sequence: SEQ ID NO: 29<br>CDR1: KSSQSLLNSANQKNYLT (SEQ ID NO: 30)<br>CDR2: WASTRES (SEQ ID NO: 31)<br><br>CDR3: QNDYNYPLT (SEQ ID NO: 32) |

From the above, it was shown that the antibody containing CDRs as described above can specifically recognize human mCRP.

Hereinafter, the following abbreviations may be used for a human denatured CRP-specific antibody.

1) Human denatured CRP-specific antibody produced from hybridoma mCRP-3C: 3C antibody
2) Human denatured CRP-specific antibody produced from hybridoma mCRP-12C: 12C antibody
3) Human denatured CRP-specific antibody produced from hybridoma mCRP-18A: 18A antibody

Example 4: Epitope analysis of human denatured CRP-specific monoclonal antibodies

(1) Peptide synthesis (part 1)

Epitopes of 3C antibodies were analyzed. First, the following peptides used for epitope analysis were synthesized. Each of the following peptides has a cysteine residue (C) added to the C-terminus of the epitope of human mCRP in order to enable immobilization to a solid phase.

1) mCRP(19):QTDMSRKAFVC (SEQ ID NO: 33)
2) mCRP(29):FPKESDTSYVC (SEQ ID NO: 34)
3) mCRP(39):SLKAPLTKPLC (SEQ ID NO: 35)
4) mCRP(49):KAFTVCLHFYC (SEQ ID NO: 36)
5) mCRP(59):TELSSTRGYSC (SEQ ID NO: 37)
6) mCRP(69):IFSYATKRQDC (SEQ ID NO: 38)
7) mCRP(79):NEILIFWSKDC (SEQ ID NO: 39)

8) mCRP(89):IGYSFTVGGSC (SEQ ID NO: 40)
9) mCRP(99):EILFEVPEVTC (SEQ ID NO: 41)
10) mCRP(109):VAPVHICTSWC (SEQ ID NO: 42)
11) mCRP(119):ESASGIVEFWC (SEQ ID NO: 43)
12) mCRP(129):VDGKPRVRKSC (SEQ ID NO: 44)
13) mCRP(139):LKKGYTVGAEC (SEQ ID NO: 45)
14) mCRP(149):ASIILGQEQDC (SEQ ID NO: 46)
15) mCRP(159):SFGGNFEGSQC (SEQ ID NO: 47)
16) mCRP(169):SLVGDIGNVNC (SEQ ID NO: 48)
17) mCRP(179):MWDFVLSPDEC (SEQ ID NO: 49)
18) mCRP(189):INTIYLGGPFC (SEQ ID NO: 50)
19) mCRP(199):SPNVLNWRALC (SEQ ID NO: 51)
20) mCRP(209):KYEVQGEVFTKPQLWPC (SEQ ID NO: 52)

(2) Epitope analysis (part 1)

The following materials were used as materials for epitope analysis.

(a) Plate: Immunoplate for ELISA (96 well) manufactured by Nunc
(b) Substrate: O.P.D tablet (SIGMA)
(c) Solid-phase buffer: 0.1 M carbonate buffer pH 9.5 (IBL)
(d) Antiserum dilution buffer: 1% BSA, 0.05% Tween-20 (Kanto Chemical Co., Inc.) in PBS
(e) Labeled antibody dilution buffer: 1% BSA, 0.05% Tween-20 in PBS
(f) Washing buffer: 0.05% Tween-20 in 0.1 M PB
(g) Substrate buffer: $K_2HPO_4$-citrate buffer (pH 5.1) (IBL)
(h) Reaction stop solution: 1 mol/L-$H_2SO_4$ (Wako Pure Chemical Industries, Ltd.)
(i) Solid phase peptide

Epitope analysis was performed by the following procedure.

(a) Peptides were immobilized on a plate by standing at 50 ng/well (50 $\mu$L/well) for 16 hours at 4°C.
(b) Solid phase peptides were washed twice with Dulbecco's PBS (D-PBS) (200 $\mu$L/well).
(c) Blocking was performed by adding 200 $\mu$L of D-PBS containing 0.1% BSA and 0.05% $NaN_3$ to each well and leaving it at 4°C overnight.
(d) Each well was washed twice with the washing buffer.
(e) The antibody was diluted to 1 $\mu$g/mL with the dilution buffer and the diluted solution was added to each well (50 $\mu$L/well) and left at 37°C for 30 minutes.
(f) Each well was washed four or more times with the washing buffer.
(g) Anti-mouse IgG (y-specific)-HRP, a labeled antibody, was added to each well (50 $\mu$L/well) and left at 37°C for 30 minutes.
(h) Each well was washed four or more times with the washing buffer.
(i) A chromogenic reaction was started by adding a substrate solution (400 $\mu$g/mL) to each well (100 $\mu$L/well), and the reaction was allowed to proceed for 15 minutes at room temperature in the dark.
(j) After 15 minutes, the reaction was stopped by addition of 1 mol/L sulfuric acid (100 $\mu$L/well), and absorbance (490 nm) was measured.

As a result, the 3C antibody exhibited reactivity to the peptide comprising EILFEVPEVT (SEQ ID NO: 2) similar to that of mCRP (Table 3). In the above (e), the same result was obtained even when the antibody concentration added to each well was changed to 5 $\mu$g/mL. When it was checked whether the 12C antibody and the 18A antibody (FIGS. 3 and 4), which are human denatured CRP-specific monoclonal antibodies, bind to a peptide comprising EILFEVPEVT (SEQ ID NO: 2) or not, they did not bind to the peptide. This indicates that these antibodies recognize an epitope different from that in the case of the 3C antibody. Accordingly, it is believed that there are not only one kind of epitope but also a plurality of kinds of epitopes of human denatured CRP-specific monoclonal antibodies.

[Table 3]

| Table 3. Epitope analysis of 3C antibody (part 1) | | | | | | | |
|---|---|---|---|---|---|---|---|
| | 19 | 29 | 39 | 49 | 59 | 69 | 79 |
| Absorbance | 0.004 | -0.001 | 0.001 | 0 | 0.001 | 0.002 | 0 |
| | | | | | | | |
| | 89 | 99 | 109 | 119 | 129 | 139 | 149 |
| Absorbance | 0 | **1.581** | -0.001 | 0.005 | 0.002 | 0.002 | 0.001 |
| | | | | | | | |
| | 159 | 169 | 179 | 189 | 199 | 209 | 159 |
| Absorbance | 0.002 | 0.001 | 0.003 | 0.007 | 0.006 | 0 | 0.002 |

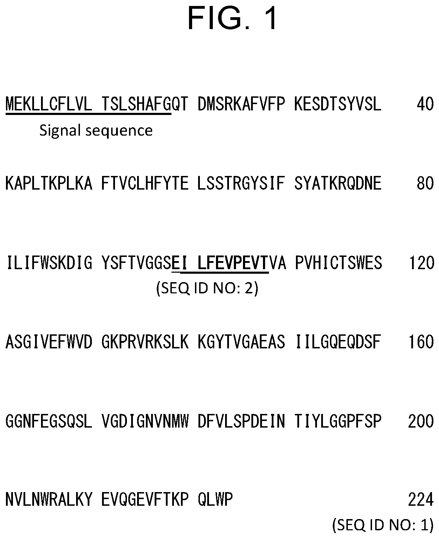

From the above, it was considered that a plurality of epitopes comprising EILFEVPEVT (SEQ ID NO: 2) (FIG. 1), which is an epitope of the 3C antibody, exist as an epitope of the human denatured CRP-specific monoclonal antibody.

(3) Peptide synthesis (part 2)

Epitopes of 3C antibodies were analyzed for replication. As the epitope, in place of the peptides of the above 1) to 20), the following longer peptides were synthesized and used.

21) mCRP(1-30):QTDMSRKAFVFPKESDTSYVSLKAPLTKPLC (SEQ ID NO: 53)
22) mCRP(31-60):KAFTVCLHFYTELSSTRGYSIFSYATKRQDC (SEQ ID NO: 54)
23) mCRP(61-90):NEILIFWSKDIGYSFTVGGSEILFEVPEVTC (SEQ ID NO: 55)
24) mCRP(91-120):VAPVHICTSWESASGIVEFWVDGKPRVRKSC (SEQ ID NO: 56)
25) mCRP(121-150):LKKGYTVGAEASIILGQEQDSFGGNFEGSQC (SEQ ID NO: 57)
26) mCRP(151-180):SLVGDIGNVNMWDFVLSPDEINTIYLGGPFC (SEQ ID NO: 58)
27) mCRP(181-206):SPNVLNWRALKYEVQGEVFTKPQLWPC (SEQ ID NO: 59)

(4) Epitope analysis (part 2)

The material for epitope analysis was the same as that in Example 4 (2). The procedure for epitope analysis was the same as that in Example 4 (2).
As a result, the 3C antibody reacted to a peptide comprising EILFEVPEVT (SEQ ID NO: 2) (Table 4). The same results were also obtained when the peptide was not directly immobilized on the solid phase but immobilized on the solid phase via bovine serum albumin (BSA).

[Table 4]

| Table 4. Epitope analysis of 3C antibody (part 2) | | | | | | | |
|---|---|---|---|---|---|---|---|
| | 1-30 | 31-60 | 61-90 | 91-120 | 121-150 | 151-180 | 181-206 |
| Absorbance | -0.001 | 0.03 | **1.982** | 0.03 | 0.017 | 0.108 | 0.006 |

From the above, it was confirmed that the epitope of the 3C antibody was EILFEVPEVT (SEQ ID NO: 2) (FIG. 1).

Example 5: Test of diseases based on denatured CRP concentration

(1) Measurement of CRP value and denatured CRP concentration

This test is approved by Tazuke Kofukai, Medical Research Institute, Kitano Hospital, Ethics Committee and Kansai Electric Power Hospital, Ethics Committee. Further, a document was presented to patients, and specimen collection was performed only from patients who agreed to the document.
As patients, patients diagnosed with a single inflammatory disease were targeted, except for patients diagnosed with double diseases. The targeted inflammatory diseases are rheumatoid arthritis (RA), adult-onset Still's disease (AOSD), vasculitis, polymyalgia rheumatica (PMR), systemic lupus erythematosus (SLE), and infectious diseases.

As a sample used for measuring the CRP value and the denatured CRP concentration, patient-derived blood plasma was used.

The CRP value was measured using N-Assay LA CRP-T Nittobo or N-Assay LA CRP-S Nittobo D-type (Nittobo Medical Co., Ltd.), which is a clinical test reagent for CRP containing an antibody against CRP.

The measurement of the denatured CRP concentration was performed by sandwich ELISA using the 12C antibody (solid phase antibody) and the 18A antibody (labeled antibody) obtained in the above Examples. Specifically, the denatured CRP concentration was measured as follows.

(a) The purified 12C antibody was prepared to 20 $\mu$g/mL with the solid-phase buffer, and immobilized on a plate by leaving at 4°C overnight using 2 $\mu$g/well (100 $\mu$L/well).
(b) The solid phase antibody was washed twice with the washing buffer.
(c) Blocking was performed by adding 200 $\mu$L of D-PBS containing 0.1% BSA and 0.05% NaN$_3$ to each well and leaving it at 4°C overnight.
(d) Each well was washed twice with the washing buffer.
(e) The specimen was diluted with the dilution buffer, and the diluted solution was added to each well (100 $\mu$L/well) and left at 37°C for 60 minutes.
(f) Each well was washed four times with the washing buffer.
(g) Fab'-labeled 18A antibody-HRP, a labeled antibody, was prepared to 0.4 $\mu$g/mL with the dilution buffer, added to each well (100 $\mu$L/well), and allowed to stand at 37°C for 30 minutes.
(h) Each well was washed five times with the washing buffer.
(i) A chromogenic reaction was started by adding a substrate solution to each well (100 $\mu$L/well), and the reaction was allowed to proceed for 30 minutes at room temperature in the dark.
(j) After 30 minutes, the reaction was stopped by addition of the reaction stop solution (100 $\mu$E/well), and absorbance (450 nm and 650 nm) was measured.
(k) The denatured CRP concentration in the specimen was calculated from the calibration curve prepared based on the measured values. Human mCRP was used as a protein for preparing a calibration curve (standard product).

The results are as follows.

[Table 5]

| Table 5. Denatured CRP concentrations and CRP values (without cut-off by CRP values) | | | |
|---|---|---|---|
| Inflammatory disease | Denatured CRP concentration | CRP value | Concentration ratio |
| RA (N=53) | 49 $\pm$ 105 | 57 $\pm$ 71 | 2.81 $\pm$ 5.76 |
| AOSD (N=13) | 612 $\pm$ 696 | 136 $\pm$ 90 | 3.94 $\pm$ 3.42 |
| Vasculitis (N=17) | 46 $\pm$ 72 | 86 $\pm$ 72 | 2.80 $\pm$ 6.06 |
| PMR (N=21) | 156 $\pm$ 226 | 104 $\pm$ 81 | 1.80 $\pm$ 2.05 |
| SLE (N=19) | 8 $\pm$ 7 | 11 $\pm$ 31 | 10.22 $\pm$ 8.19 |
| Infectious disease (N=32) | 305 $\pm$ 372 | 168 $\pm$ 99 | 2.05 $\pm$ 2.29 |

The symbol "N" indicates the number of patients tested.
The "denatured CRP concentration" indicates the average concentration $\pm$ SD (ng/mL) in blood.
The "CRP value" indicates the mean concentration $\pm$ SD ($\mu$g/mL) in blood.
The "concentration ratio" indicates a ratio of the average concentration (ng/mL) of the denatured CRP/CRP value ($\mu$g/mL) (hereinafter, the same shall apply).
Next, denatured CRP-positive patients whose CRP value exceeded the reference value were extracted. As a reference value of the CRP value, $\geq$ 50 $\mu$g/mL was adopted. Further, $\geq$ 150 ng/mL was adopted as a reference value for denatured CRP-positive.

[Table 6]

| Table 6. Number of denatured CRP-positive patients with CRP values exceeding reference values | | |
|---|---|---|
| Inflammatory disease | Number of patients (percentage) | |
| | CRP value ≥ 50 mg/mL | CRP value ≥ 5 mg/100 mL and denatured CRP concentration ≥ 150 ng/mL |
| RA (N=53) | 22/53 | 4/53 (about 1%) |
| AOSD (N=13) | 11/13 | 9/13 (about 69%) |
| Vasculitis (N=17) | 12/17 | 0/17 (0%) |
| PMR (N=21) | 16/21 | 7/21 (about 33%) |
| SLE (N=19) | 1/19 | 0/19 (0%) |
| Infectious disease (N=32) | 32/32 | 16/32 (50%) |
| Total (N=155) | 94/155 | 36/155 (about 23%) |

Next, for patients with CRP values exceeding the above reference values, the denatured CRP concentration and CRP value, and the ratio therebetween were extracted, and the p value was calculated by Dunn-Bonferroni test between adult-onset Still's disease (AOSD) and other diseases to examine statistically significant differences.

It is to be noted that for SLE, the denatured CRP concentration was overwhelmingly low, and only one patient with a CRP value exceeding 50 μg/mL was found, so the subsequent analysis was omitted. However, since AOSD and SLE are fundamentally different in both the denatured CRP concentration and the CRP value, it is understood that AOSD can be easily differentiated from SLE by measuring the denatured CRP concentration and/or CRP value regardless of whether the subsequent analysis was performed or not.

[Table 7]

| Table 7. Denatured CRP concentration and CRP value (with cut-off by CRP value) | | | |
|---|---|---|---|
| Inflammatory disease | Denatured CRP concentration | CRP value | Ratio |
| RA (N=22) | 93 ± 148 | 124 ± 65 | 0.88 ± 1.35 |
| AOSD (N=11) | 722 ± 702 | 157 ± 83 | 4.62 ± 3.26 |
| Vasculitis (N=12) | 21 ± 27 | 116 ± 65 | 0.15 ± 0.17 |
| PMR (N=16) | 203 ± 241 | 134 ± 69 | 1.60 ± 1.92 |
| Infectious disease (N=32) | 305 ± 372 | 168 ± 99 | 2.05 ± 2.29 |

[Table 8]

| Table 8. Statistically significant differences | | |
|---|---|---|
| Inflammatory disease | p value | |
| | Denatured CRP concentration | Ratio |
| AOSD (N=11) vs. Vasculitis (N=12) | 0.00005 > | 0.0005 > |
| AOSD (N=11) vs. RA (N=22) | 0.0005 > | 0.001 > |
| AOSD (N=11) vs. PMR (N=16) | 0.055 | 0.050 > |

Example 6: Test of diseases based only on denatured CRP concentration

The number of cases was increased, and the denatured CRP concentration and the CRP value (without cut-off by CRP value) were measured in the same manner as in the method described in Example 5.

The results are as follows.

[Table 9]

Table 9. Denatured CRP concentrations and CRP values (without cut-off by CRP values)

| Inflammatory disease | Denatured CRP concentration | CRP value | Concentration ratio |
|---|---|---|---|
| RA (N=55) | 42 ± 97 | 56 ± 70 | 1.89 ± 5.21 |
| AOSD (N=20) | 477 ± 582 | 149 ± 93 | 3.34 ± 2.81 |
| Vasculitis (N=18) | 88 ± 175 | 93 ± 65 | 42.12 ± 172.72 |
| PMR (N=23) | 162 ± 214 | 101 ± 75 | 1.33 ± 1.71 |
| SLE (N=19) | 8 ± 7 | 11 ± 31 | 10.22 ± 8.19 |
| Infectious disease (N=40) | 281 ± 342 | 173 ± 99 | 1.89 ± 2.13 |

The symbol "N" indicates the number of patients tested.

The "denatured CRP concentration" indicates the average concentration ± SD (ng/mL) in blood.

The "CRP value" indicates the mean concentration ± SD ($\mu$g/mL) in blood.

The "concentration ratio" indicates a ratio of the average concentration (ng/mL) of the denatured CRP/CRP value ($\mu$g/mL) (hereinafter, the same shall apply).

Finally, the denatured CRP concentration and the CRP value, and the ratio therebetween were extracted, the p value was calculated by Dunn-Bonferroni test between adult-onset Still's disease (AOSD) and other diseases, and the statistically significant difference was examined.

It is to be noted that for SLE, the denatured CRP concentration was overwhelmingly low, and thus the subsequent analysis was omitted. However, since AOSD and SLE are fundamentally different in both the denatured CRP concentration and the CRP value, it is understood that AOSD can be easily differentiated from SLE by measuring the denatured CRP concentration and/or CRP value regardless of whether the subsequent analysis was performed or not.

[Table 10]

Table 10. Statistically significant differences

| Inflammatory disease | p value | |
|---|---|---|
| | Denatured CRP concentration | Ratio |
| AOSD (N=20) vs. Vasculitis (N=18) | 0.05 > | 0.05 > |
| AOSD (N=20) vs. RA (N=55) | 0.001 > | 0.001 > |
| AOSD (N=20) vs. PMR (N=23) | 0.005 > | 0.05 > |

(ASD)

As ASD, AOSD was used for evaluation. In AOSD and infectious diseases, blood plasma denatured CRP concentrations were higher than in other inflammatory diseases (Tables 5 to 10). Interestingly, the denatured CRP concentration in blood plasma from AOSD patients was significantly higher than the previously reported concentration of the denatured CRP in blood plasma from patients with myocardial infarction (20.96 ng/mL) (Wang et al., Atherosclerosis, 2015, vol. 239, p. 343-349) and significantly higher than the previously reported concentration of the denatured CRP in blood plasma from psoriasis, eczema, and urticaria (skin-related autoimmune disease) patients (35.4 ng/mL, 30.0 ng/mL and 59.8 ng/mL, respectively) (Zhang et al., Frontiers in Immunology, 2018, vol. 9, Article 511). That is, interestingly and unexpectedly, the denatured CRP concentration in the body fluid derived from AOSD patients was higher than the previously reported denatured CRP concentration known to the present inventors. An infectious disease may often be easy to diagnose depending on its symptoms. Further, since infectious diseases can be definitely diagnosed by identifying a pathogen (for example, viruses and microorganisms) (for example, gene/genome detection), AOSD is often easily distinguished as an infectious disease. As described above, considering that AOSD and infectious diseases are easily distinguished from each other, a high denatured CRP concentration is useful for testing AOSD.

Accordingly, it was confirmed that the level of the denatured CRP concentration and the level of the CRP denaturation degree associated therewith [for example, a high ratio (denatured CRP concentration/CRP value)] are useful as indicators of ASD such as AOSD.

(Vasculitis)

In Vasculitis and SLE, denatured CRP concentrations in blood plasma were lower than in other inflammatory diseases (Tables 5 and 9). It can be understood that since SLE has a low CRP value in blood plasma (Tables 5 and 9), the denatured CRP concentration tends to be low in conformity with the low CRP value. Since SLE can be tested by measuring markers such as double-stranded DNA, urinary protein, and an autoantibody, vasculitis can be easily differentiated from SLE. Since SLE shows a low CRP value unlike vasculitis (Tables 5 and 9), vasculitis can be easily differentiated from SLE. Considering that vasculitis and SLE are easily differentiated from each other as described above, a low denatured CRP concentration is useful as an index of vasculitis.

Further, vasculitis showed the lowest ratio (average denatured CRP concentration/average CRP concentration) due to the high CRP value and significantly low denatured CRP concentration in inflammatory diseases (Tables 5 to 10).

Accordingly, it was confirmed that the low level of denatured CRP concentration and the low level of CRP denaturation degree associated therewith [for example, a low ratio (denatured CRP concentration/CRP value)] are useful as indicators of vasculitis.

(Differentiation between ASD and Vasculitis)

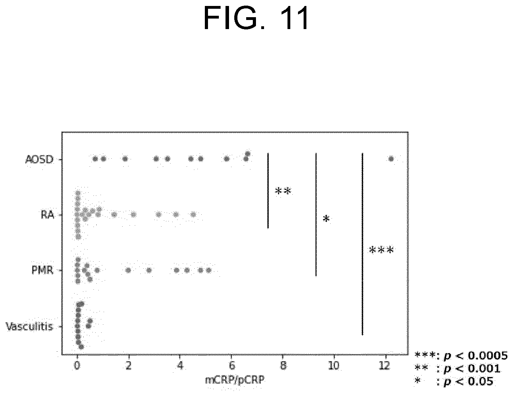

AOSD and vasculitis showed highly statistically significant differences in both denatured CRP concentration and CRP denaturation degree (Tables 8 and 10, and FIG. 11). This indicates that the CRP denaturation degree is excellent as an index for differentiation between ASD such as AOSD and vasculitis. Accordingly, it was confirmed that the CRP denaturation degree is useful for differentiation between ASD and vasculitis.

(Differentiation between ASD and RA and PMR)

In ASD, RA, and PMR, pyrexia, joint pain, and muscle pain are common symptoms. CRP also often shows moderate to high elevation, which makes it difficult to make a differential diagnosis. The appearance of the eruption, which is characteristic of AOSD, helps in the differentiation, but since 20 to 30% of AOSD does not have an eruption, differentiation becomes more difficult in that case. Further, so-called seronegative RA (RF-negative and CCP-negative) exists in about 30% of RA, and a specific marker has not been found in PMR at the present time. There are no specific markers for vasculitis except for some (ANCA-associated vasculitis). Accordingly, differentiation of ASD such as AOSD from serologically negative vasculitis, serologically negative RA, and PMR is clinically very important. From this viewpoint, a differentiation method using a denatured CRP concentration or a denatured CRP concentration/CRP is expected as a clinically very useful means.

Example 7: Preparation and analysis of humanized antibodies and chimeric antibody

(1) Preparation of humanized antibodies and chimeric antibody

Humanized antibodies and a chimeric antibody that share the complementarity determining regions (CDRs) 1 to 3 in the heavy chain (HC) and light chain (LC) of the human denatured CRP-specific monoclonal antibody (3C antibody) obtained in the previous Examples were prepared, and their binding capacities were analyzed.

First, from the amino acid sequences of the heavy chain and the light chain of the 3C antibody, CDRs 1 to 3 were identified based on the Kabat and IMGT numbering schemes. The identified CDRs 1 to 3 were grafted into variable regions based on the human immunoglobulin heavy chain variable gene (IGHV) and the human immunoglobulin kappa variable gene (IGKV).

Next, the heavy chain variable region (VH) and the light chain variable region (VL) after transplantation were cloned into expression vectors having the amino acid sequences of the constant regions of IgG1 (G1m17,1) and IgK1 (Km3), respectively, to produce humanized antibodies containing a heavy chain (HCs 1 to 5) obtained by transplanting CDRs 1 to 3 in the heavy chain of the 3C antibody (mouse antibody) into a human antibody and a light chain (LCs 1 to 3) obtained by transplanting CDRs 1 to 3 in the light chain of the 3C antibody into a human antibody.

A chimeric antibody containing a heavy chain (HC 0) in which a heavy chain variable region of a 3C antibody (mouse antibody) was linked to a heavy chain constant region of a human antibody and a light chain (LC 0) in which a light chain variable region of a 3C antibody was linked to a light chain constant region of a human antibody was also prepared.

Information on the heavy chain (HCs 0 to 5) and light chain (LCs 0 to 3) of each of the produced antibodies is shown in Table 11.

[Table 11]

| | SEQ ID NO | | Position of specific region in amino acid sequence | | | |
|---|---|---|---|---|---|---|
| Table 11. Relationship between heavy chain and light chain of chimeric antibody and humanized antibody, and specific region | | | | | | |
| | Nucleotide sequence | Amino acid sequence | Variable region | CDR1 | CDR2 | CDR3 |
| HC0 | SEQ ID NO: 60 | SEQ ID NO: 61 | Positions 20-133 | Positions 45-54 | Positions 69-85 | Positions 116-122 |
| HC1 | SEQ ID NO: 62 | SEQ ID NO: 63 | Positions 20-133 | Positions 45-54 | Positions 69-85 | Positions 116-122 |
| HC2 | SEQ ID NO: 64 | SEQ ID NO: 65 | Positions 20-133 | Positions 45-54 | Positions 69-85 | Positions 116-122 |
| HC3 | SEQ ID NO: 66 | SEQ ID NO: 67 | Positions 20-133 | Positions 45-54 | Positions 69-85 | Positions 116-122 |
| HC4 | SEQ ID NO: 68 | SEQ ID NO: 69 | Positions 20-133 | Positions 45-54 | Positions 69-85 | Positions 116-122 |
| HC5 | SEQ ID NO: 70 | SEQ ID NO: 71 | Positions 201-133 | Positions 45-54 | Positions 69-85 | Positions 116-122 |
| LC0 | SEQ ID NO: 72 | SEQ ID NO: 73 | Positions 21-132 | Positions 44-59 | Positions 75-81 | Positions 114-122 |
| LC1 | SEQ ID NO: 74 | SEQ ID NO: 75 | Positions 21-132 | Positions 44-59 | Positions 75-81 | Positions 114-122 |
| LC2 | SEQ ID NO: 76 | SEQ ID NO: 77 | Positions 21-132 | Positions 44-59 | Positions 75-81 | Positions 114-122 |
| LC3 | SEQ ID NO: 78 | SEQ ID NO: 79 | Positions 21-132 | Positions 44-59 | Positions 75-81 | Positions 114-122 |

(2) Analysis of humanized antibodies and chimeric antibody

The binding ability of each of the prepared antibodies was analyzed. The expression vector was introduced into CHO cells and cultured, and then the expressed antibody was purified from the culture supernatant using an AKTA™ design chromatography system. The binding ability of the obtained 10 kinds of purified antibodies was analyzed by two kinds of methods.

First, the binding ability of the purified antibody was analyzed using Octet Systems utilizing Bio-Layer Interferometry (BLI). The binding ability of the humanized antibody (HCx/LCx) was evaluated in percentage with the binding ability of the chimeric antibody (HC 0/LC 0) as 100%.

Next, the binding ability of the purified antibody was analyzed by Biacore™.

As a result, it was confirmed that all of the 10 kinds of purified antibodies showed high binding ability (Table 12).

[Table 12]

| Table 12. Binding ability of purified antibodies | | | | | | |
|---|---|---|---|---|---|---|
| | HC/LC* | Analysis of binding ability | | | | |
| | | BLI | Biacore™ | | | |
| | | | $ka\,(M^{-1}s^{-1})$ global fit | $kd\,(s^{-1})$ separate fit | $K_D$ (M) | % of $R_{max}$ theor |
| Chimeric antibody | HC0/LC0 | 100 | 3.78 E +03 | < 4.19 E-06 | < 1.11 E-09 | 379 |

(continued)

| Table 12. Binding ability of purified antibodies | | | | | | |
|---|---|---|---|---|---|---|
| | HC/LC* | Analysis of binding ability | | | | |
| | | BLI | Biacore™ | | | |
| | | | ka (M$^{-1}$s$^{-1}$) global fit | kd (s$^{-1}$) separate fit | $K_D$ (M) | % of R$_{max}$ theor |
| Humanized antibody 1 | HC1/LC3 | 132.13 | 3.88 E +03 | < 4.19 E-06 | < 1.08 E-09 | 417 |
| Humanized antibody 2 | HC2/LC1 | 100.69 | 2.97 E +03 | 7.58 E-06 | 2.55 E-09 | 235 |
| Humanized antibody 3 | HC2/LC3 | 109.05 | 3.04 E +03 | < 4.19 E-06 | < 1.38 E-09 | 226 |
| Humanized antibody 4 | HC3/LC1 | 96.42 | 3.77 E +03 | < 4.19 E-06 | < 1.11 E-09 | 353 |
| Humanized antibody 5 | HC3/LC3 | 100.93 | 3.68 E +03 | < 4.19 E-06 | < 1.14 E-09 | 400 |
| Humanized antibody 6 | HC4/LC1 | 109.67 | 2.79 E +03 | < 4.19 E-06 | < 1.50 E-09 | 309 |
| Humanized antibody 7 | HC4/LC2 | 79.71 | 2.27 E +03 | < 4.19 E-06 | < 1.84 E-09 | 228 |
| Humanized antibody 8 | HC4/LC3 | 92.08 | 2.92 E +03 | < 4.19 E-06 | < 1.44 E-09 | 375 |
| Humanized antibody 9 | HC5/LC1 | 92.87 | 3.39 E +03 | < 4.19 E-06 | < 1.24 E-09 | 306 |
| Humanized antibody 10 | HC5/LC3 | 116.93 | 3.69 E +03 | < 4.19 E-06 | < 1.14 E-09 | 392 |
| * The combination of heavy chain (HC) and (light chain) in an antibody is shown. | | | | | | |

According to at least one embodiment described above, it is possible to assist clinical diagnosis.

Although some embodiments of the present invention have been described, these embodiments have been presented as examples, and are not intended to limit the scope of the invention. These novel embodiments can be implemented in various other forms, and various omissions, substitutions, and changes can be made without departing from the gist of the invention. These embodiments and modifications thereof are included in the scope and gist of the invention, and are included in the invention described in the claims and the equivalent scope thereof.

[Sequence Listing]

**Claims**

1. A method for testing adult Still's disease, comprising the following (1) and (2):

   (1) measuring a denatured CRP concentration in a blood sample collected from a subject; and
   (2) comparing the measured denatured CRP concentration and/or an index value based on the denatured CRP concentration with a reference value.

2. The method according to claim 1, wherein the subject is a subject contracting an inflammatory disease or a subject suspected of contracting an inflammatory disease.

3. The method according to claim 1 or 2, wherein the subject is a subject contracting a disease other than an infectious disease or a subject suspected of contracting a disease other than an infectious disease.

4. The method according to any one of claims 1 to 3, which is a method for differentiating adult Still's disease from vasculitis, rheumatoid arthritis, polymyalgia rheumatica, or systemic lupus erythematosus.

5. The method according to any one of claims 1 to 4, wherein the index value is a value calculated from a relational expression between the denatured CRP concentration and a CRP value.

6. The method according to any one of claims 1 to 5, wherein the value calculated from the relational expression between the denatured CRP concentration and the CRP value is a value calculated from a relative relational expression between the denatured CRP concentration and the CRP value.

7. The method according to any one of claims 1 to 6, wherein the subject is a subject whose CRP value is measured.

8. The method according to claim 7, wherein the subject exhibits a CRP value higher than the reference value.

9. The method according to any one of claims 1 to 6, wherein the subject is a subject whose CRP value is not measured, and
the method further comprises a step of measuring the CRP value in a blood sample collected from the subject.

10. The method according to claim 9, further comprising selecting and subjecting a subject whose measured CRP value is higher than the reference value to the comparing step.

11. The method according to any one of claims 1 to 10, wherein the denatured CRP concentration is measured using a denatured CRP-specific antibody.

12. The method according to claim 11, wherein the denatured CRP concentration is measured using the denatured CRP-specific antibody selected from the group consisting of the following (1) to (3), or a combination thereof:

    (1) an antibody comprising the following (1a) and (1b):

       (1a) an antibody heavy chain containing a variable region comprising an amino acid sequence having an identity of 90% or more to the amino acid sequence consisting of the amino acid residues at positions 20 to 133 in the amino acid sequence of SEQ ID NO: 4, and
       (1b) an antibody light chain containing a variable region comprising an amino acid sequence having an identity of 90% or more to the amino acid sequence consisting of the amino acid residues at positions 20 to 131 in the amino acid sequence of SEQ ID NO: 9;

    (2) an antibody comprising the following (2a) and (2b) :

       (2a) an antibody heavy chain containing a variable region comprising an amino acid sequence having an identity of 90% or more to the amino acid sequence consisting of the amino acid residues at positions 20 to 141 in the amino acid sequence of SEQ ID NO: 14, and
       (2b) an antibody light chain containing a variable region comprising an amino acid sequence having an identity of 90% or more to the amino acid sequence consisting of the amino acid residues at positions 21 to 133 in the amino acid sequence of SEQ ID NO: 19; and

    (3) an antibody comprising the following (3a) and (3b) :

       (3a) an antibody heavy chain containing a variable region comprising an amino acid sequence having an identity of 90% or more to the amino acid sequence consisting of the amino acid residues at positions 20 to 141 in the amino acid sequence of SEQ ID NO: 24, and
       (3b) an antibody light chain containing a variable region comprising an amino acid sequence having an identity of 90% or more to the amino acid sequence consisting of the amino acid residues at positions 21 to 133 in the amino acid sequence of SEQ ID NO: 29.

13. A denatured CRP-specific antibody selected from the group consisting of the following (1) to (3):

(1) an antibody comprising the following (1a) and (1b):

(1a) an antibody heavy chain containing a variable region comprising an amino acid sequence having an identity of 90% or more to the amino acid sequence consisting of the amino acid residues at positions 20 to 133 in the amino acid sequence of SEQ ID NO: 4, and
(1b) an antibody light chain containing a variable region comprising an amino acid sequence having an identity of 90% or more to the amino acid sequence consisting of the amino acid residues at positions 20 to 131 in the amino acid sequence of SEQ ID NO: 9;

(2) an antibody comprising the following (2a) and (2b) :

(2a) an antibody heavy chain containing a variable region comprising an amino acid sequence having an identity of 90% or more to the amino acid sequence consisting of the amino acid residues at positions 20 to 141 in the amino acid sequence of SEQ ID NO: 14, and
(2b) an antibody light chain containing a variable region comprising an amino acid sequence having an identity of 90% or more to the amino acid sequence consisting of the amino acid residues at positions 21 to 133 in the amino acid sequence of SEQ ID NO: 19; and

(3) an antibody comprising the following (3a) and (3b) :

(3a) an antibody heavy chain containing a variable region comprising an amino acid sequence having an identity of 90% or more to the amino acid sequence consisting of the amino acid residues at positions 20 to 141 in the amino acid sequence of SEQ ID NO: 24, and
(3b) an antibody light chain containing a variable region comprising an amino acid sequence having an identity of 90% or more to the amino acid sequence consisting of the amino acid residues at positions 21 to 133 in the amino acid sequence of SEQ ID NO: 29.

**14.** A kit for testing adult Still's disease, comprising a denatured CRP-specific antibody.

# FIG. 1

<u>MEKLLCFLVL TSLSHAFG</u>QT DMSRKAFVFP KESDTSYVSL    40
       Signal sequence

KAPLTKPLKA FTVCLHFYTE LSSTRGYSIF SYATKRQDNE    80

ILIFWSKDIG YSFTVGGS<u>EI LFEVPEVT</u>VA PVHICTSWES    120
           (SEQ ID NO: 2)

ASGIVEFWVD GKPRVRKSLK KGYTVGAEAS IILGQEQDSF    160

GGNFEGSQSL VGDIGNVNMW DFVLSPDEIN TIYLGGPFSP    200

NVLNWRALKY EVQGEVFTKP QLWP                  224
                                  (SEQ ID NO: 1)

FIG. 2

mCRP–3C

OD (490 nm)

Antibody concentration (ng/mL)

Inhibition (-)

mCRP Inhibition

pCRP Inhibition

FIG. 3

mCRP-12C

Legend:
- Inhibition (-)
- mCRP Inhibition
- PCRP Inhibition

Y-axis: OD (490 nm)
X-axis: Antibody concentration (ng/mL): 1000, 500, 250, 125, 62.5, 31.3, 15.6, 7.8, 3.9, 2.0, 1.0, 0.5

EP 4 293 360 A1

FIG. 4

mCRP-18A

EP 4 293 360 A1

# FIG. 5

M E W S W V F L F L L S V T A G V Q S Q     20

Signal sequence

V Q L Q Q S G A E L V R P G A S V K L S     40

C K A L D Y T F T D Y E M H W V K Q T P     60

CDR1 (SEQ ID NO: 5)

V H G L E W I G A I H P G R G G T A Y N     80

CDR2 (SEQ ID NO: 6)

Q K F K G K A T L T A D K S S S T A Y M     100

E L S S L T S E D S G V Y Y C T S Y H G     120

CDR3 (SEQ ID NO: 7)

G Y W G Q G T T L T V S S A K T T P P S     140

V Y P L A P G C G D T T G S S V T L G C     160
L V K G Y F P E S V T V T W N S G S L S     180
S S V H T F P A L L Q S G L Y T M S S S     200
V T V P S S T W P S Q T V T C S V A H P     220
A S S T T V D K K L E P S G P I S T I N     240
P C P P C K E C H K C P A P N L E G G P     260
S V F I F P P N I K D V L M I P L T P K     280
V T C V V V D V S E D D P D V Q I S W F     300
V N N V E V H T A Q T Q T H R E D Y N S     320
T I R V V S T L P I Q H Q D W M S G K E     340
F K C K V N N K D L P S P I E R T I S K     360
I K G L V R A P Q V Y I L P P P A E Q L     380
S R K D V S L T C L V V G F N P G D I S     400
V E W T S N G H T E E N Y K D T A P V L     420
D S D G S Y F I Y S K L N M K T S K W E     440
K T D S F S C N V R H E G L K N Y Y L K     460
K T I S H S P G K     469

(SEQ ID NO: 4)

# FIG. 6

<u>M  K  L  P  V  R  L  L  V  L  M  F  W  I  P  A  A  S  S</u>  D       20
      Signal sequence

S  C  <u>R  S  S  Q  S  L  V  H  S  N  E  N  T  Y  L  L</u>  W  Y     60
           CDR1 (SEQ ID NO: 10)

L  Q  K  P  G  Q  S  P  K  L  L  I  Y  <u>K  V  S  N  R  F  S</u>    80
                    CDR2 (SEQ ID NO: 11)

G  V  P  D  R  F  S  G  S  G  S  G  T  D  F  T  L  K  I  S    100

R  V  E  A  E  D  L  G  V  Y  F  C  <u>S  Q  T  T  H  V  P  W</u>    120
                  CDR3 (SEQ ID NO: 12)

<u>T</u>  F  G  G  G  T  K  L  E  I  K  R  A  D  A  A  P  T  V  S    140

I  F  P  P  S  S  E  Q  L  T  S  G  G  A  S  V  V  C  F  L    160
N  N  F  Y  P  K  D  I  N  V  K  W  K  I  D  G  S  E  R  Q    180
N  G  V  L  N  S  W  T  D  Q  D  S  K  D  S  T  Y  S  M  S    200
S  T  L  T  L  T  K  D  E  Y  E  R  H  N  S  Y  T  C  E  A    220
T  H  K  T  S  T  S  P  I  V  K  S  F  N  R  N  E  C    238
                      (SEQ ID NO: 9)

# FIG. 7

<u>M K C S W I I F F L M A V V T G V N S</u> E    20
<center>Signal sequence</center>

V Q L Q Q S G A E L V K P G A S V K L S    40

C T A S G F N I K <u>D Y Y I H</u> W V K Q R T    60
<center>CDR1 (SEQ ID NO: 15)</center>

D Q G L E W I G <u>R I D P E D D E T K Y A</u>    80
<center>CDR2 (SEQ ID NO: 16)</center>

<u>P K F Q G</u> K A T I I T D T S S N T A Y L    100

H L S S L T S A D T A V Y Y C A S <u>R I Y</u>    120
<center>CDR3 (SEQ ID NO: 17)</center>

<u>Y Y D I K G L F D Y</u> W G Q G T T L T V S    140

S A K T T P P S V Y P L A P G S A A Q T    160
N S M V T L G C L V K G Y F P E P V T V    180
T W N S G S L S S G V H T F P A V L Q S    200
D L Y T L S S S V T V P S S T W P S Q T    220
V T A T L P T R P A A P R W T R K L C P    240
G I V V V S F A Y V Q S Q K Y H L S S S    260
S P P K P K D V L T I T L T P K V T C V    280
V V D I S K D D P E V Q F S W F V D D V    300
E V H T A Q T K P R E E Q I N S T F R S    320
V S E L P I M H Q D W L N G K E F K C R    340
V N S A A F P A P I E K T I S K T K G R    360
P K A P Q V Y T I P P P K E Q M A K D K    380
V S L T C M I T N F F P E D I T V E W Q    400
W N G Q P A E N Y K N T Q P I M D T D G    420
S Y F V Y S K L N V Q K S N W E A G N T    440
F T C S V L H E G L H N H H T E K S L S    460
H S P G K    465

<div align="right">(SEQ ID NO: 14)</div>

# FIG. 8

```
M  E  S  Q  T  Q  V  L  M  S  L  L  F  W  V  S  G  T  C  G      20
                        Signal sequence

D  I  V  M  T  Q  A  P  S  S  L  T  V  T  A  G  E  K  V  T      40

M  T  C  K  S  S  Q  S  L  L  N  S  G  N  Q  K  N  Y  L  T      60
            CDR1 (SEQ ID NO: 20)

W  S  Q  Q  K  P  G  Q  P  P  K  L  L  I  Y  W  A  S  T  R      80
                                    CDR2 (SEQ ID NO: 21)

E  S  G  V  P  D  R  F  T  G  S  G  S  G  T  D  F  T  L  T     100

I  S  S  V  Q  A  E  D  L  A  V  Y  Y  C  Q  N  D  Y  N  Y     120
                              CDR3 (SEQ ID NO: 22)

P  I  T  F  G  A  G  T  K  L  E  L  K  R  A  D  A  A  P  T     140

V  S  I  F  P  P  S  S  E  Q  L  T  S  G  G  A  S  V  V  C     160
F  L  N  N  F  Y  P  K  D  I  N  V  K  W  K  I  D  G  S  E     180
R  Q  N  G  V  L  N  S  W  T  D  Q  D  S  K  D  S  T  Y  S     200
M  S  S  T  L  T  L  T  K  D  E  Y  E  R  H  N  S  Y  T  C     220
E  A  T  H  K  T  S  T  S  P  I  V  K  S  F  N  R  N  E  C     240
                                        (SEQ ID NO: 19)
```

# FIG. 9

M K C S W I I F F L M A V V T G V N S E    20
Signal sequence

V Q L Q Q S G A E F V K P G T S V K L S    40

C T A S G F N I K D Y Y I H W V K Q R T    60
CDR1 (SEQ ID NO: 25)

E Q G L E W I G R I D P E D G E T K Y A    80
CDR2 (SEQ ID NO: 26)

P K F Q G K A T I T A D A S S N T A Y L    100

Q L S S L T S E D T A V Y Y C V S R M Y    120
CDR3 (SEQ ID NO: 27)

Y Y G S Q G L F D F W G Q G T T L T V S    140

S A K T T A P S V Y P L A P V C G G T T    160
G S S V T L G C L V K G Y F P E P V T L    180
T W N S G S L S S G V H T F P A L L Q S    200
G L Y T L S S S V T V T S N T W P S Q T    220
I T C N V A H P A S S T K V D K K I E P    240
R V P I T Q N P C P P L K E C P P C A A    260
P D L L G G P S V F I F P P K I K D V L    280
M I S L S P M V T C V V V D V S E D D P    300
D V Q I S W F V N N V E V H T A Q T Q T    320
H R E D Y N S T L R V V S A L P I Q H Q    340
D W M S G K E F K C K V N N R A L P S P    360
I E K T I S K P R G P V R A P Q V Y V L    380
P P P A E E M T K K E F S L T C M I T G    400
F L P A E I A V D W T S N G R T E Q N Y    420
K N T A T V L D S D G S Y F M Y S K L R    440
V Q K S T W E R G S L F A C S V V H E G    460
L H N H L T T K T I S H S P G K    476

(SEQ ID NO: 24)

# FIG. 10

| | | | | | | | | | | | | | | | | | | | | |
|---|---|---|---|---|---|---|---|---|---|---|---|---|---|---|---|---|---|---|---|---|
| M | E | S | Q | T | Q | V | L | M | S | L | L | F | W | V | S | G | T | C | G | 20 |

Signal sequence

| | | | | | | | | | | | | | | | | | | | | |
|---|---|---|---|---|---|---|---|---|---|---|---|---|---|---|---|---|---|---|---|---|
| D | I | V | M | T | Q | S | P | S | S | L | T | V | T | A | G | E | K | V | T | 40 |

| | | | | | | | | | | | | | | | | | | | | |
|---|---|---|---|---|---|---|---|---|---|---|---|---|---|---|---|---|---|---|---|---|
| M | G | C | K | S | S | Q | S | L | L | N | S | A | N | Q | K | N | Y | L | T | 60 |

CDR1 (SEQ ID NO: 30)

| | | | | | | | | | | | | | | | | | | | | |
|---|---|---|---|---|---|---|---|---|---|---|---|---|---|---|---|---|---|---|---|---|
| W | Y | R | Q | K | P | G | Q | P | P | K | L | L | I | Y | W | A | S | T | R | 80 |

CDR2 (SEQ ID NO: 31)

| | | | | | | | | | | | | | | | | | | | | |
|---|---|---|---|---|---|---|---|---|---|---|---|---|---|---|---|---|---|---|---|---|
| E | S | G | V | P | D | R | F | T | G | S | G | S | G | T | D | F | T | L | T | 100 |

| | | | | | | | | | | | | | | | | | | | | |
|---|---|---|---|---|---|---|---|---|---|---|---|---|---|---|---|---|---|---|---|---|
| I | S | S | V | Q | A | E | D | L | A | V | Y | Y | C | Q | N | D | Y | N | Y | 120 |

CDR3 (SEQ ID NO: 32)

| | | | | | | | | | | | | | | | | | | | | |
|---|---|---|---|---|---|---|---|---|---|---|---|---|---|---|---|---|---|---|---|---|
| P | L | T | C | G | A | G | T | K | L | D | L | K | R | A | D | A | A | P | T | 140 |
| V | S | I | F | P | P | S | S | E | Q | L | T | S | G | G | A | S | V | V | C | 160 |
| F | L | N | N | F | Y | P | K | D | I | N | V | K | W | K | I | D | G | S | E | 180 |
| R | Q | N | G | V | L | N | S | W | T | D | Q | D | S | K | D | S | T | Y | S | 200 |
| M | S | S | T | L | T | L | T | K | D | E | Y | E | R | H | N | S | Y | T | C | 220 |
| E | A | T | H | K | T | S | T | S | P | I | V | K | S | F | N | R | N | E | C | 240 |

(SEQ ID NO: 29)

# FIG. 11

## INTERNATIONAL SEARCH REPORT

| International application No. |
|---|
| **PCT/JP2022/005590** |

**A. CLASSIFICATION OF SUBJECT MATTER**

*G01N 33/68*(2006.01)i; *C07K 16/18*(2006.01)i; *C12N 15/13*(2006.01)i; *G01N 33/53*(2006.01)i
FI: G01N33/68 ZNA; G01N33/53 D; C07K16/18; C12N15/13

According to International Patent Classification (IPC) or to both national classification and IPC

**B. FIELDS SEARCHED**

Minimum documentation searched (classification system followed by classification symbols)

G01N33/68; C12N15/13; G01N33/53

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); MEDLINE/EMBASE (STN); 医中誌Web

**C. DOCUMENTS CONSIDERED TO BE RELEVANT**

| Category* | Citation of document, with indication, where appropriate, of the relevant passages | Relevant to claim No. |
|---|---|---|
| X | CN 102703388 A (WANG, Junhong) 03 October 2012 (2012-10-03)<br>abstract, claims, paragraphs [0045]-[0073], examples 1-5 | 13 |
| A | MITROVIC, Stephane, FAUTREL, Bruno. New Markers for Adult-Onset Still's Disease. Joint Bone Spine. 18 May 2017, 2018, vol. 85, pp. 285-293<br>abstract, 4.1, table 3 | 1-14 |
| A | WO 2019/208542 A1 (NAGASAKI UNIVERSITY) 31 October 2019 (2019-10-31)<br>entire text, all drawings | 1-14 |
| A | US 2015/0072022 A1 (UNIVERSITY OF MASSACHUSETTS) 12 March 2015 (2015-03-12)<br>entire text, all drawings | 1-14 |
| A | US 2006/0057642 A1 (KIEFER, Charles R.) 16 March 2006 (2006-03-16)<br>entire text, all drawings | 1-14 |
| A | JP 2006-115716 A (MATSUSHITA ELECTRIC INDUSTRIAL COMPANY LIMITED) 11 May 2006 (2006-05-11)<br>entire text, all drawings | 1-14 |

☐ Further documents are listed in the continuation of Box C.  ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **05 April 2022** | **19 April 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

International application No.

**PCT/JP2022/005590**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed:

       ☑ in the form of an Annex C/ST.25 text file.

       ☐ on paper or in the form of an image file.

   b. ☐ furnished together with the international application under PCT Rule 13ter.1(a) for the purposes of international search only in the form of an Annex C/ST.25 text file.

   c. ☐ furnished subsequent to the international filing date for the purposes of international search only:

       ☐ in the form of an Annex C/ST.25 text file (Rule 13ter.1(a)).

       ☐ on paper or in the form of an image file (Rule 13ter.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

## INTERNATIONAL SEARCH REPORT

International application No.

**PCT/JP2022/005590**

| Box No. III | Observations where unity of invention is lacking (Continuation of item 3 of first sheet) |
|---|---|

This International Searching Authority found multiple inventions in this international application, as follows:

Invention 1: Claims 1-12 and 14

Claims 1-12 have the special technical feature of a method for examining adult-onset Still's disease, comprising (1) and (2), and are thus classified as invention 1.

Claim 14 has the corresponding technical feature of examination of adult-onset Still's disease by utilizing modified CRP, and thus is classified as invention 1.

Invention 2: Claim 13

Claim 13 and claim 1 classified as invention 1 share the technical feature of modified CRP.

However, this technical feature does not make a contribution over the prior art in light of the content disclosed in document 1, and thus cannot be said to be a special technical feature. Moreover, there are no other same or corresponding special technical features among these inventions.

Furthermore, claim 13 is not depend on claim 1. In addition, claim 13 is not substantially identical to or similarly closely related to any of the claims classified as invention 1.

Therefore, claim 13 cannot be classified as invention 1.

Document 1: CN 102703388 A (WANG, Junhong) 03 October 2012 (2012-10-03), abstract, claims, paragraphs [0045]-[0073], examples 1-5

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**   ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
**Information on patent family members**

| International application No. |
| :--- |
| **PCT/JP2022/005590** |

| Patent document cited in search report | Publication date (day/month/year) | Patent family member(s) | Publication date (day/month/year) |
| :--- | :--- | :--- | :--- |
| CN 102703388 A | 03 October 2012 | (Family: none) | |
| WO 2019/208542 A1 | 31 October 2019 | JP 2019-190976 A | |
| US 2015/0072022 A1 | 12 March 2015 | (Family: none) | |
| US 2006/0057642 A1 | 16 March 2006 | WO 2006/031592 A1 whole document | |
| JP 2006-115716 A | 11 May 2006 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004189665 A

**Non-patent literature cited in the description**

- **YAYOI NAGAI.** *The Japanese Journal of Dermatology,* 2006, vol. 116 (4), 429-436
- **NOBORU HAGINO.** *The Journal of the Japanese Society of Internal Medicine,* 2009, vol. 98 (10), 2 421-2431
- **KOHEI NAGASAWA.** *The Journal of the Japanese Society of Internal Medicine,* 2010, vol. 99 (10), 2460-2466
- **YAMAGUCHI et al.** *The Journal of Rheumatology,* 1992, vol. 19, 424-430
- **WANG et al.** *Atherosclerosis,* 2015, vol. 239, 343-349
- **ZHANG et al.** *Frontiers in Immunology,* 2018, vol. 9